(19) Europäisches Patentamt
European Patent Office
Office européen des brevets

(11) **EP 3 118 758 B1**

(12) **FASCICULE DE BREVET EUROPEEN**

(45) Date de publication et mention
de la délivrance du brevet:
**27.02.2019 Bulletin 2019/09**

(51) Int Cl.:
***G06F 19/00*** (2018.01)

(21) Numéro de dépôt: **16305626.0**

(22) Date de dépôt: **31.05.2016**

(54) **PROCEDE D'EXPLOITATION D'UN GISEMENT D'HYDROCARBURES CONTENANT DES COMPOSES ORGANO-SOUFRES AU MOYEN D'UN MODELE THERMO-CINETIQUE ET D'UNE SIMULATION DE RESERVOIR COMPOSITIONNELLE**

AUSBEUTUNGSVERFAHREN EINES KOHLENWASSERSTOFFLAGERS, DAS ORGANOSCHWEFELVERBINDUNGEN ENTHÄLT, MIT HILFE EINES THERMOKINETISCHEN MODELLS UND EINER SIMULATION DER ZUSAMMENSETZUNG DES LAGERS

METHOD FOR EXPLOITING A HYDROCARBON DEPOSIT CONTAINING ORGANO-SULPHUR COMPOUNDS USING A THERMOKINETIC MODEL AND A COMPOSITIONAL RESERVOIR SIMULATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.06.2015 FR 1555087**

(43) Date de publication de la demande:
**18.01.2017 Bulletin 2017/03**

(73) Titulaire: **IFP Energies nouvelles**
**92500 Rueil-Malmaison (FR)**

(72) Inventeur: **AYACHE, Simon**
**92000 Nanterre (FR)**

(74) Mandataire: **IFP Energies nouvelles**
**Rond-point de l'échangeur de Solaize**
**BP3**
**69230 Solaize (FR)**

(56) Documents cités:
**EP-A1- 2 775 092    FR-A1- 2 892 817**

- MERCHAN S. ET AL: "Upscaling Using a Non-Uniform Coarsened Grid With Optimum Power Average", JOURNAL OF CANADIAN PETROLEUM TECHNOLOGY, vol. 46, no. 07, 1 juillet 2007 (2007-07-01), page 21, XP055156396, ISSN: 0021-9487, DOI: 10.2118/07-07-01
- MAMAGHANI M. ET AL: "Development of a refinement criterion for adaptive mesh refinement in steam-assisted gravity drainage simulation", COMPUTATIONAL GEOSCIENCES, vol. 15, no. 1, 16 juin 2010 (2010-06-16), pages 17-34, XP055271109, NL ISSN: 1420-0597, DOI: 10.1007/s10596-010-9192-4

## Description

L'invention concerne le domaine de l'exploration pétrolière, et plus particulièrement le domaine de l'exploitation d'un gisement d'hydrocarbures contenant des composés organo-soufrés, par un procédé thermique tel qu'un procédé d'injection de vapeur.

Lors de l'exploitation de réservoirs de bruts lourds par un procédé d'injection de vapeur, il se produit un phénomène d'aquathermolyse, qui peut générer de l'hydrogène sulfuré ($H_2S$). En effet ce type de réservoir peut contenir des teneurs en soufre souvent élevées. Les procédés thermiques permettent, par apport de calories et augmentation de la température, de réduire la viscosité des bruts lourds et ainsi de les rendre productibles.

L'aquathermolyse est définie dans la présente description comme un ensemble de réactions physico-chimiques entre une roche imprégnée d'huile brute (ou de bitume) et de la vapeur d'eau, à des températures entre 200°C et 300°C.

L'hydrogène sulfuré est un gaz à la fois extrêmement corrosif et très toxique, voire létal au-delà d'une certaine concentration. Aussi, prévoir la concentration en $H_2S$ du gaz produit lors de la récupération assistée par injection de vapeur aide, d'une part, à réduire les coûts de production en adaptant les matériaux de complétion et les dispositifs de traitement de gaz, en optimisant les conditions d'opérations, et d'autre part, à éviter des émissions dangereuses pour l'homme et l'environnement.

Un problème technique est la prédiction de la quantité d'$H_2S$ générée selon la nature du brut, les conditions de réservoir et les conditions d'injection de vapeur. Si l'on veut prédire le risque de production d'$H_2S$ grâce à un modèle de réservoir (utilisé par les simulateurs d'écoulements), un modèle cinétique de genèse de l'hydrogène sulfuré est nécessaire.

Par ailleurs, les réactions physico-chimiques associées au phénomène d'aquathermolyse engendrent une modification de la composition de l'huile lourde. Il peut donc être avantageux que le modèle cinétique utilisé pour la prédiction de la genèse de l'hydrogène sulfuré puisse également prédire les variations de la composition de l'huile dans le réservoir. Cette capacité permet en effet de prévoir les effets du procédé d'injection de vapeur sur la qualité de l'huile produite.

## État de la technique

Les documents suivants seront cités au cours de la description :

Barroux, C., Lamoureux-Var, V., Flauraud, E., 2013. Forecasting of H2S Production due to Aquathermolysis Reactions, Paper SPE 164317, presented at the SPE Middle East Oil and Gas Show and Conference, Manam, Bahrain.

Belgrave J.D.M., Moore R.G., Ursenbach R.G, (1997), "Comprehensive kinetic models for the aquathermolysis of heavy oils", Journal of Canadian Petroleum Technology, v 36, n 4, p 38-44.

Boduszynski, M.M. 1987. Composition of Heavy Petroleums. 1. Molecular Weight, Hydrogen Deficiency, and Heteroatom Concentration as a Function of Atmospheric Equivalent Boiling Point up to 1400 °F (760 °C). Energy & Fuels, 1, 2-11.

Coats, K. H. 1980. In-Situ Combustion Model. SPE Journal, December, 533-554.

Merdrignac, I. and Espinat D. 2007. Physicochemical Characterization of Petroleum Fractions: the State of the Art. Oil & Gas Science and Technology - Rev. IFP, 62, 1, 7-32.

Crookston, R. B., Culham, W. E., Chen, W. H. 1979. A Numerical Simulation Model Recovery Processes for Thermal Recovery Processes. SPE 6724, SPE J., Feb., 37-58

Fan T. and Buckley, J., Rapid and Accurate SARA Analysis of Medium Gravity Crude Oils, Energy Fuels, 2002, 16 (6), pp 1571-1575.

Lamoureux-Var, V. and Lorant, F. (2005a).Barroux, C., (2013), Using Geochemistry to Address H2S Artificial Formation as a Result of Production Risk due to Steam Injection for EOR: a Compositional Kinetic Approach. in Oil Sands. Paper 13HOCC-P-412-SPE 97810, SPE/PS-CIM/CHOA International Thermal Operations and Heavy Oil Symposium, 1-3 NovemberConference, Calgary, Alberta, Canada, 11-14 June.

Lamoureux-Var, V. and Lorant, F. (2005b). Expérimental evaluation of H2S yields in reservoir rocks submitted to steam injection. Paper D08, 13th European Symposium on Improved Oil Recovery, EAGE, Budapest, Hungary, 25 - 27 April.

Peng, D. Y., and Robinson, D. B. 1976. A New Two-Constant Equation of State. Industrial and Engineering Chemistry Fundamentals, 15, 59-64.

Søreide, I. and Whitson, C. H. 1992. Peng-Robinson Predictions for Hydrocarbons CO2, N2, and H2S with Pure Water and NaCl Brine. Fluid Phase Equilibria, 77, 217-240.

On connaît de la demande de brevet FR 2892817 (US 11/588365) une méthode permettant de construire un modèle cinétique pour estimer la masse d'hydrogène sulfuré produite par aquathermolyse d'une roche contenant de l'huile brute. Cette méthode utilise une représentation compositionnelle massique de l'huile brute par classes de composés chimiques de type S.A.R.A. (description des hydrocarbures en quatre fractions massiques ou pseudo-constituants : les Saturés, les Aromatiques, les Résines et les Asphaltènes ; voir par exemple (Fan and Buckley, 2002)) et décrit l'évolution de la répartition du soufre dans lesdites fractions massiques de l'huile et une fraction dite insoluble (qui représente le solide recevant une partie du soufre initialement détenu dans l'huile au cours des réactions d'aquathermolyse). La méthode proposée repose sur un schéma réactif élémentaire sur l'élément soufre, obtenu par bilans massiques sur l'élément soufre réparti au sein des différentes fractions (SARA et solide). Toutefois ce schéma n'est pas utilisable dans une simulation de réservoir. En effet, la simulation de réservoir requiert des informations sur des pseudo-constituants décrits chacun par une molécule, et non uniquement par une masse comme dans la demande de brevet FR 2892817 (US 11/588365).

On connaît également la demande FR 3002969 (US 14/200682) qui vise à transformer le modèle cinétique basé sur la distribution du soufre de la demande FR 2892817 (US 11/588365) en un modèle cinétique compositionnel moléculaire. Ce modèle est développé dans le but de prédire la production d'$H_2S$ résultant des réactions d'aquathermolyse dans le contexte de la simulation de réservoir. Toutefois ce modèle présente un certain nombre d'inconvénients. Tout d'abord, le bilan atomique sur le soufre, supposé respecté par ce procédé (puisque le modèle cinétique compositionnel est construit autour du schéma réactif sur l'élément soufre développé dans la demande FR 2892817 (US 11/588365)), ne l'est pas en pratique. En effet, la mise en oeuvre de cette méthode montre que les coefficients stoechiométriques dérivés à partir du schéma réactif du soufre ne permettent pas de reproduire les résultats observés en laboratoire, notamment les variations en masse des fractions SARA et de l'$H_2S$ au cours du temps. Les coefficients stoechiométriques doivent alors être modifiés afin de caler aux observations des expériences d'aquathermolyse. Le bilan atomique sur le soufre, qui est pourtant à la base de ce modèle cinétique compositionnel, n'est alors lui-même plus respecté une fois le modèle cinétique calibré. Par ailleurs, le modèle cinétique développé dans cette demande, basé uniquement sur l'élément soufre, ne permet pas de respecter les bilans atomiques moyens des éléments atomiques, autres que le soufre, constitutifs des pseudo-molécules représentatives des différentes fractions SARA, à savoir du carbone (C), de l'hydrogène (H) et de l'oxygène (O). Ces limitations ont pour conséquence le fait que le schéma réactif décrit dans la demande FR 3002969 (US 14/200682) ne permet pas d'intégrer des données expérimentales, telles que les résultats d'analyses élémentaires menées en laboratoire, qui pourraient pourtant agir comme contraintes à respecter en reliant le nombre d'atomes des éléments constitutifs de chacun des pseudo-constituants à son nombre d'atomes de carbone. Par ailleurs, le modèle cinétique de la demande FR 3002969 (US 14/200682) ne permet pas de prendre en compte l'élément oxygène, ce qui empêche la prédiction de l'évolution de l'eau ($H_2O$) et du gaz carbonique ($CO_2$) lors des réactions d'aquathermolyse. Pourtant l'$H_2O$ est un réactif essentiel des réactions d'aquathermolyse, sans lequel les réactions ne peuvent avoir lieu. La possibilité de prendre en compte l'eau dans le schéma réactif compositionnel du modèle d'aquathermolyse apparaît donc importante lorsqu'une précision accrue des résultats de simulation de réservoir est souhaitée, et afin de renforcer l'aspect prédictif du modèle. Enfin, le bilan massique entre réactifs et produits de chacune des réactions sur lesquelles repose la demande FR 3002969 (US 14/200682) est bien respecté lors de la calibration du modèle, mais cette contrainte n'est satisfaite qu'en ajustant les coefficients stoechiométriques de la fraction des saturés. En effet, le procédé décrit dans la demande de brevet FR 3002969 (US 14/200682), qui repose sur le schéma réactif du soufre du brevet FR 2892817 (US 11/588365), déduit le coefficient stoechiométrique associé aux saturés en effectuant un simple bilan massique entre réactifs et produits sur chaque réaction. Or, comme l'eau n'est pas incorporée au schéma réactif compositionnel, tout comme de nombreuses espèces chimiques, un simple bilan de masse sur les réactions du modèle ne peut pas permettre de déterminer un coefficient stoechiométrique approprié pour les saturés. Dès lors, au sein du modèle de la demande FR 3002969 (US 14/200682), les saturés jouent le rôle de variable d'ajustement afin de respecter le bilan massique de chacune des réactions du schéma réactif de l'aquathermolyse.

L'objet de l'invention concerne un procédé d'exploitation d'un gisement d'hydrocarbures contenant des composés organo-soufrés au moyen d'un modèle thermo-cinétique et d'une simulation de réservoir compositionnelle. Le modèle cinétique construit via le procédé selon l'invention permet de mieux respecter la théorie des phénomènes physiques et chimiques impliqués dans une réaction d'aquathermolyse (notamment le respect des bilans atomiques), mais aussi de prendre en compte certaines mesures expérimentales disponibles (telles que des analyses élémentaires), et ainsi de contribuer à une quantification plus précise des émissions d'hydrogène sulfuré.

**Le procédé selon l'invention**

Ainsi, la présente invention concerne un procédé d'exploitation un gisement souterrain d'hydrocarbures contenant des composés organo-soufrés. Le procédé selon l'invention comporte les étapes suivantes :

A. on détermine une quantité d'hydrogène sulfuré ($H_2S$) produite au cours du temps par un phénomène d'aquathermolyse induit par un procédé thermique tel qu'une injection de vapeur d'eau dans ledit gisement, à partir d'une représentation maillée dudit gisement, à partir de mesures expérimentales réalisées sur au moins un échantillon desdits hydrocarbures et/ou un échantillon de roche dudit gisement, en mettant en oeuvre les étapes suivantes :

  i. on construit un schéma réactif élémentaire représentatif d'un bilan de matière sur l'élément soufre à partir d'au moins une représentation compositionnelle des hydrocarbures, ladite représentation décrivant lesdits hydrocarbures en différents pseudo-constituants, d'au moins un pseudo-constituant solide, d'au moins un constituant $H_2S$, et de coefficients pseudo-stoechiométriques relatifs auxdits pseudo-constituants et constituants ;

  ii. on construit un modèle cinétique à partir d'au moins un système de réactions simulant ledit phénomène d'aquathermolyse, ledit schéma réactif, de bilans atomiques relatifs à chacune desdites réactions dudit système de réactions, lesdits bilans portant au moins sur les atomes de soufre, de carbone et d'hydrogène, et ledit système de réactions étant fonction de coefficients stoechiométriques relatifs au moins auxdits pseudo-constituants et constituants ;

  iii. on construit un modèle thermodynamique des pseudo-constituant de ladite représentation compositionnelle ;

  iv. on calibre un modèle thermo-cinétique, constitué dudit modèle cinétique et dudit modèle thermodynamique, à partir desdites mesures expérimentales ;

  v. on détermine ladite quantité d'hydrogène sulfuré ($H_2S$) produite en réalisant une simulation de réservoir compositionnelle, au moyen d'un simulateur thermique compositionnel et réactif, ledit simulateur mettant en oeuvre ledit modèle thermo-cinétique, et au moyen de ladite représentation maillée ;

B. on détermine des conditions d'exploitation dudit gisement en fonction de ladite quantité d'hydrogène sulfuré ;

C. on produit lesdits hydrocarbures en appliquant lesdites conditions d'exploitation.

Avantageusement, ladite représentation compositionnelle des hydrocarbures peut être constituée des pseudo-constituants suivants : les hydrocarbures saturés, les hydrocarbures aromatiques, les résines et les asphaltènes.

Préférentiellement, lesdites mesures expérimentales peuvent consister en des expériences d'aquathermolyse simulées en laboratoire.

Selon un mode de réalisation de l'invention, l'étape ii) peut être réalisée selon les étapes suivantes :

a) on définit un système de réactions décrivant ledit phénomène d'aquathermolyse dans ledit gisement, ledit système étant fonction au moins desdits pseudo-constituants et au moins des constituants $H_2S$ et $H_2O$, et desdits coefficients stoechiométriques relatifs à chacun desdits pseudo-constituants et constituants ;

b) on définit un premier système d'équations établissant, pour chacune desdites réactions, des bilans atomiques pour les éléments atomiques H, C, S et O ;

c) on définit un deuxième système d'équations reliant lesdits coefficients pseudo-stoechiométriques dudit schéma réactif auxdits coefficients stoechiométriques dudit système de réactions, la somme desdits coefficients pseudo-stoechiométriques dudit schéma réactif étant égales à 1 ;

d) on détermine une expression desdits coefficients stoechiométriques dudit système de réactions en résolvant conjointement lesdits systèmes d'équations.

Préférentiellement, on peut définir un troisième système d'équations reliant le nombre d'atomes de soufre, de carbone et d'oxygène en fonction du nombre d'atomes de carbone pour chacun desdits pseudo-constituants et constituants.

Avantageusement, on peut relier ledit nombre d'atomes de soufre, de carbone et d'oxygène en fonction dudit nombre d'atomes de carbone pour chacun desdits pseudo-constituants via des ratios déterminés par des analyses élémentaires réalisées sur des produits d'aquathermolyse simulées en laboratoire.

Procédé selon l'une des revendications précédentes, dans lequel à l'étape iii), on calibre au moins des paramètres cinétiques dudit modèle thermo-cinétique.

Procédé selon l'une des revendications 1 à 6, dans lequel à l'étape iii), on calibre des paramètres cinétiques dudit modèle thermo-cinétique et lesdits coefficients pseudo-stoechiométriques dudit schéma réactif.

Selon un mode de réalisation de l'invention, on peut déterminer en sus à l'étape A) une évolution au cours du temps de la quantité de chacun desdits pseudo-constituants de ladite représentation compositionnelle.

Selon un mode de mise en oeuvre de l'invention, on peut déterminer lesdites conditions d'exploitation en adaptant des matériaux de complétion et/ou des dispositifs de traitement de gaz en fonction de ladite quantité d'hydrogène sulfuré.

Selon un mode de réalisation de l'invention, lesdites conditions d'exploitation peuvent consister à modifier des conditions d'injection de la vapeur de façon à minimiser ladite quantité d'hydrogène sulfuré.

Selon un mode de mise en oeuvre de l'invention, on peut déterminer lesdites conditions d'exploitation de façon à maintenir une production d'hydrogène sulfuré inférieure à une teneur maximale légale.

En outre, l'invention concerne un produit programme d'ordinateur téléchargeable depuis un réseau de communication et/ou enregistré sur un support lisible par ordinateur et/ou exécutable par un processeur, comprenant des instructions de code de programme pour la mise en oeuvre du procédé selon la description ci-dessus, lorsque ledit programme est exécuté sur un ordinateur.

**Présentation sommaire des figures**

D'autres caractéristiques et avantages du procédé selon l'invention, apparaîtront à la lecture de la description ci-après d'exemples non limitatifs de réalisations, en se référant aux figures annexées et décrites ci-après.

La Figure 1 présente l'évolution au cours du temps de la masse d'$H_2S$ rapportée à la masse totale initiale des pseudo-constituants SARA, obtenue par une mise en oeuvre de la présente invention et par méthode expérimentale.

Les Figures 2A, 2B, 2C et 2D présentent l'évolution au cours du temps de la masse des pseudo-constituants SAT, ARO, RES et ASP respectivement, rapportée à leur masse initiale, obtenue par une mise en oeuvre de la présente invention et par méthode expérimentale.

La Figure 3 présente la production simulée d'$H_2S$ en fonction du temps obtenue par une mise en oeuvre de la présente invention et par des mesures in situ.

Les Figures 4A et 4B présentent l'évolution de la fraction molaire des pseudo-constituants ASP, ARO, RES, SAT et du constituant $H_2S$ au cours du temps, obtenue par une mise en oeuvre de la présente invention.

**Description détaillée du procédé selon l'invention**

Les définitions suivantes sont utilisées au cours de la description de l'invention :

- hydrocarbures : utilisé au sens large, comme souvent en ingénierie de réservoir, il s'agit à la fois des hydrocarbures au sens strict, composés exclusivement d'atomes d'hydrogène et de carbone (fraction des Saturés dans le cas de la représentation compositionnelle SARA), et d'autres composés organiques comprenant en plus des hétéro-éléments tels que l'oxygène et le soufre (fractions des Aromatiques soufrés, des Résines, et des Asphaltènes dans le cas de la représentation compositionnelle SARA).

- constituant : il s'agit d'une espèce moléculaire telle que l'hydrogène sulfuré ($H_2S$), le méthane ($CH_4$), l'eau ($H_2O$) ;

- pseudo-constituant : il s'agit d'un mélange d'espèces moléculaires assimilable à une espèce moléculaire unique pour le problème traité ;

- élément atomique : il s'agit d'une espèce élémentaire atomique comme le soufre S, le carbone C, l'hydrogène H, etc ;

- schéma réactif du soufre : il s'agit d'un modèle cinétique élémentaire construit sur la répartition du soufre élémentaire entre le constituant H$_2$S, les pseudo-constituants d'une représentation compositionnelle des hydrocarbures (telle que la représentation SARA) et un pseudo-constituant solide. Ce schéma réactif du soufre permet de décrire la genèse d'H$_2$S en fonction du soufre contenu dans les pseudo-constituants d'une représentation compositionnelle des hydrocarbures, lorsque la roche réservoir est mise en contact avec de l'eau à une température comprise entre 150°C et 340°C, en fonction de la température et de la durée de contact entre la roche réservoir et l'eau. Ce schéma vise à représenter la façon dont l'élément soufre se répartit lors du craquage des composés organo-soufrés, contenus dans les hydrocarbures au sens large, engendré par la réaction d'aquathermolyse. A noter qu'un schéma réactif élémentaire sur l'élément soufre est non utilisable en l'état dans une simulation de réservoir, qui utilise des informations sur des constituants/pseudo-constituants de type moléculaire, et non des informations sur des éléments atomiques.

- coefficients stoechiométriques : il s'agit de coefficients indiquant les proportions moléculaires entre les différentes espèces intervenant dans l'équilibre d'une réaction chimique.

- coefficients pseudo-stoechiométriques : il s'agit de coefficients indiquant les proportions massiques entre les différentes espèces intervenant dans une réaction chimique. On a recours à des coefficients pseudo-stoechiométriques dans le cas de pseudo-constituants dont on ne connait pas ou mal la masse molaire, et pour lesquels on écrit alors les équations chimiques en proportions massiques.

La présente invention concerne un procédé, et son utilisation, pour modéliser la production d'hydrogène sulfuré (H$_2$S) induite par des réactions se produisant dans un gisement souterrain d'hydrocarbures lorsque ce gisement est soumis à un procédé de récupération thermique, en particulier à un procédé d'injection de vapeur, les réactions étant alors dues à un phénomène d'aquathermolyse.

Le procédé selon l'invention comporte au moins les étapes suivantes :

### 1. Détermination d'une quantité d'hydrogène sulfuré (H$_2$S) produite

#### 1.1. Construction d'un schéma réactif du soufre

#### 1.2. Construction d'un modèle cinétique

#### 1.3. Construction d'un modèle thermodynamique

#### 1.4. Calibration du modèle thermo-cinétique

#### 1.5. Mise en oeuvre de la simulation de réservoir

### 2. Détermination des conditions d'exploitation en fonction de la quantité d'hydrogène sulfuré

### 3. Production des hydrocarbures

Pour sa mise en oeuvre, la présente invention requiert de disposer :

- **de mesures expérimentales réalisées sur au moins un échantillon de pétrole ou de roche du gisement étudié :**
  En premier lieu, des mesures expérimentales sont nécessaires pour la mise en oeuvre de l'étape de calibration du modèle thermo-cinétique du procédé selon l'invention. Pour ce faire, on réalise des expériences d'aquathermolyse en laboratoire, qui consistent à faire chauffer un échantillon de roche du gisement ou de pétrole avec de l'eau, à une température constante et sous pression contrôlée pendant une durée déterminée. Afin de calibrer les paramètres cinétiques (facteurs de fréquence, énergies d'activation) du modèle thermo-cinétique et, si besoin (c'est-à-dire si ces coefficients ne sont pas déjà connus), les coefficients pseudo-stoechiométriques du schéma réactif du soufre, on peut répéter plusieurs expériences d'aquathermolyse, à différentes températures et pour différentes durées. Puis, à l'issue de ces expériences d'aquathermolyse, on mesure la masse des différents constituants/pseudo-constituants intervenant dans les réactions d'aquathermolyse, ainsi que leur teneur massique en soufre atomique. On peut en déduire un premier bilan de masse sur l'ensemble des constituants/pseudo-constituants ; il s'agit de la somme des masses des constituants/pseudo-constituants après réaction divisée par la somme des masses des constituants/pseudo-constituants mis dans le système avant réaction. On peut en déduire ensuite un deuxième bilan de masse sur la distribution massique du soufre atomique sur l'ensemble des constituants/pseudo-constituants ; il s'agit de la somme sur l'ensemble des constituants/pseudo-constituants des ratios entre la masse de soufre atomique dans un constituant/pseudo-constituant et la masse de soufre total impliqué dans le système. De ces deux calculs de bilan de masse, on peut déduire, d'une part, l'évolution de la proportion massique des constituants/pseudo-constituants au cours du temps et pour différentes températures, et d'autre part, l'évolution de la distribution massique du soufre atomique parmi les constituants/pseudo-constituants au cours du temps et pour différentes températures. Ces expériences répétées permettent de calibrer les paramètres cinétiques du modèle thermo-cinétique, mais aussi les coefficients pseudo-stoechiométriques du schéma réactif du soufre.

En second lieu, des mesures expérimentales peuvent être utiles comme contraintes pour la construction du modèle cinétique du procédé selon l'invention, ou encore pour réduire le nombre d'inconnues de ce modèle cinétique. Pour ce faire, on peut réaliser une analyse élémentaire des produits d'expériences d'aquathermolyse effectuées en laboratoire. Bien connue du spécialiste en géochimie, l'analyse élémentaire d'un pseudo-constituant consiste à quantifier sa proportion massique en éléments atomiques tels que le carbone, l'hydrogène, le soufre, etc. Ces mesures donnent accès aux rapports entre espèces atomiques élémentaires S/C, H/C et O/C pour ces pseudo-constituants.

- **d'un simulateur de réservoir thermique compositionnel et réactif :** un simulateur de réservoir est un programme numérique, exécuté sur un ordinateur, qui est utilisé pour prédire l'écoulement des fluides (l'huile, la saumure et le gaz) à travers le milieu poreux du réservoir ainsi que la production au moyen de puits de ces fluides en surface. Dans le cas d'un simulateur thermique, la température dans le réservoir n'est pas constante mais varie par exemple en fonction de la conduction thermique dans le réservoir ou des changements de phases des différents constituants si la simulation est également compositionnelle. En effet, dans le cadre d'une simulation compositionnelle, les phases hydrocarbures (gaz et huile) sont définies comme un ensemble de constituants ou pseudo-constituants. Les propriétés de ces phases (densité, viscosité...) sont alors calculées en fonction de leur composition. Enfin, un simulateur compositionnel est qualifié de réactif s'il contient un module de réactions chimiques qui autorise les constituants/pseudo-constituants définis à réagir chimiquement entre eux. Un exemple d'un tel logiciel est le logiciel PUMAFLOW (IFP Energies nouvelles, France).

- **une représentation maillée du gisement étudié :** appelée aussi « modèle de réservoir » ou « géomodèle », il s'agit d'une sorte de maquette du sous-sol construite dans le but de décrire aussi précisément que possible la structure, les propriétés pétrophysiques, les propriétés des fluides, etc., du gisement étudié. Cette maquette est généralement représentée sur un ordinateur, et consiste en un maillage ou grille, chacune des mailles de cette grille comportant une ou plusieurs valeurs de propriétés pétrophysiques (porosité, perméabilité, saturation...). Un modèle de réservoir se doit de vérifier autant que possible l'ensemble des données collectées sur le terrain : les données de diagraphie mesurées le long des puits, les mesures réalisées sur des échantillons de roche prélevés dans les puits, les données déduites de campagnes d'acquisition sismique, les données de production comme les débits d'huile, d'eau, les variations de pression etc. Le spécialiste en simulation de réservoir a pleine connaissance de méthodes pour construire une telle représentation maillée d'un gisement.

Les principales étapes de la présente invention sont détaillées ci-après.

## 1. Détermination d'une quantité d'hydrogène sulfuré (H$_2$S) produite

L'objectif de cette étape est d'estimer, par simulation de réservoir compositionnelle, la quantité d'hydrogène sulfuré (H$_2$S) qui serait produite si l'on utilisait un procédé thermique pour exploiter un réservoir souterrain imprégné d'huile ou de bitume contenant des composés organo-soufrés (c'est-à-dire imprégné d'hydrocarbures au sens large).

### 1.1. Construction d'un schéma réactif du soufre

Au cours de cette étape, on construit un schéma réactif élémentaire représentatif d'un bilan de matière sur l'élément soufre, dit aussi schéma réactif du soufre. Le schéma réactif du soufre selon l'invention requiert de définir une représentation compositionnelle des hydrocarbures.

Selon un mode de réalisation préféré de la présente invention, on utilise, comme dans la demande de brevet FR 2892817 (US 11/588365), une représentation compositionnelle par classes de composés chimiques de type S.A.R.A., décrite par exemple dans (Fan and Buckley, 2002). Cette représentation, classiquement utilisée dans l'industrie, consiste à décrire les hydrocarbures en quatre fractions : les hydrocarbures Saturés, les hydrocarbures Aromatiques, les Résines et les Asphaltènes, et à fournir la fraction massique de chacune de ces fractions. Plus précisément, la représentation SARA est constituée de :

- un pseudo-constituant représentant la classe des composés non soufrés ; ce pseudo-constituant est assimilé à la classe des Saturés, et est désigné par SAT,

- au moins un pseudo-constituant représentant la classe des Aromatiques ; ce pseudo-constituant est désigné par ARO,

- un ou plusieurs pseudo-constituant(s) représentant la classe des Résines ; ces pseudo-composants sont désignés par $RES_1$, $RES_2$, .., $RES_p$,

- un ou plusieurs pseudo-constituant(s) représentant la classe asphalténique ; ces pseudo-composants sont désignés par $ASP_1$, $ASP_2$, .., $ASP_q$.

Ces pseudo-constituants permettent de simuler les phases fluides, ou appelées à se fluidifier, notamment par effet de température, comme dans le cas de l'injection de vapeur. Par ailleurs, chacun des pseudo-constituants soufrés {ARO, $SLD_1$, $SLD_2$, .., $SLD_s$, $RES_1$, $RES_2$, .., $RES_p$, $ASP_1$, $ASP_2$, .., $ASP_q$} peut être assimilé à une pseudo-molécule de formule générique $R_{nR}S_{nS}$ où S désigne l'atome Soufre de masse $M_S$ et R un ensemble d'atomes considéré comme un pseudo-élément atomique unique de masse $M_R$, $n_S$, $n_R$ désignant respectivement les nombres d'atomes S et de pseudo-éléments R dans la pseudo-molécule de masse molaire *MW*.

Par ailleurs, le schéma réactif du soufre selon l'invention prend également au moins en compte :

- un ou plusieurs pseudo-constituants de type solide, comme du coke désignés par $SLD_1$, $SLD_2$, .., $SLD_s$, ces pseudo-constituants solides étant au nombre de Ns. Le pseudo-constituant de type solide est considéré pour représenter le solide recevant une partie du soufre détenu dans les hydrocarbures au cours des réactions d'aquathermolyse. Ce pseudo-constituant solide est un produit des réactions d'aquathermolyse et décrit le pyrobitume généré par ces réactions.

- un constituant $H_2S$.

De plus, le schéma réactif du soufre selon l'invention est fonction de coefficients pseudo-stœchiométriques relatifs aux pseudo-constituants et constituants impliqués dans ledit schéma réactif.

Selon un mode préféré de mise en oeuvre de la présente invention, on utilise le schéma réactif élémentaire représentatif d'un bilan de matière sur l'élément soufre mis en oeuvre dans la demande de brevet FR2892817, et qui décrit la répartition de tout le soufre dans les différents pseudo-constituants précédemment mentionnés sur la base des hypothèses suivantes :

- on estime que la fraction des saturés ne contient pas de soufre ;

- on estime que le soufre contenu dans la fraction des résines donne naissance à de l'hydrogène sulfuré et s'incorpore en partie dans les fractions des solides et des aromatiques ;

- on estime que le soufre contenu dans la fraction des asphaltènes donne naissance à de l'hydrogène sulfuré et s'incorpore en partie dans les fractions des solides et des aromatiques ;

- on suppose en outre que le soufre dans les asphaltènes et le soufre dans les résines n'interagissent pas ;

- de plus, on considère que plusieurs réactions coexistent en parallèle au sein de chaque fraction, ces réactions étant caractérisées par des constantes de temps différentes.

Ainsi, selon ce mode de réalisation, le craquage des composants organiques sulfurés présents dans les classes des résines et des asphaltènes est supposé être la principale contribution à la production d'$H_2S$. Autrement dit, les pseudo-constituants des classes asphalthènes et résines jouent le rôle de réactants dans le schéma réactif élémentaire du soufre selon ce mode de réalisation.

Selon un mode préférentiel de mise en oeuvre de l'invention, on utilise le schéma réactif élémentaire représentatif d'un bilan de matière sur l'élément soufre décrit dans la demande de brevet FR 289281, en ne considérant que deux pseudo-constituants représentant la classe des résines (notés RES1 et RES2), deux pseudo-constituants représentant la classe des asphaltènes (notés ASP1 et ASP2), et un seul pseudo-constituant solide (noté SLD). De plus, pour chaque classe de réactants (RESi et ASPi, avec i=1,2), on suppose que les deux pseudo-constituants associés génèrent chacun une réaction avec une cinétique de réaction propre. On aboutit ainsi à la construction d'un schéma réactif du soufre à quatre réactions tel que décrit selon un système de réactions de la forme :

$$S^{RES1} \rightarrow a_{S1H2S}\, S^{H2S} + a_{S1ARO}\, S^{ARO} + a_{S1SLD}\, S^{SLD}$$

$$S^{RES2} \rightarrow a_{S2H2S}\, S^{H2S} + a_{S2ARO}\, S^{ARO} + a_{S2SLD}\, S^{SLD} \qquad (1)$$

$$S^{ASP1} \rightarrow a_{S3H2S}\, S^{H2S} + a_{S3ARO}\, S^{ARO} + a_{S3SLD}\, S^{SLD}$$

$$S^{ASP2} \rightarrow a_{S4H2S}\, S^{H2S} + a_{S4ARO}\, S^{ARO} + a_{S4SLD}\, S^{SLD},$$

où $S^{H2S}$, $S^{RES1}$ et $S^{RES2}$, $S^{ASP1}$ et $S^{ASP2}$, $S^{ARO}$, $S^{SLD}$, désignent respectivement le soufre hébergé par l'$H_2S$, par les pseudo-constituants RES1 et RES2 représentant la classe des résines, par les pseudo-constituants ASP1 et ASP2 représentant la classe des asphaltènes, par le pseudo-constituant ARO représentant la classe des aromatiques, et par le pseudo-constituant SLD de type solide, les coefficients $a_{SrConstk}$ (avec $1 \leq r \leq 4$ et $Const_{1 \leq k \leq 3}$={H2S, ARO, SLD}) étant des coefficients pseudo-stoechiométriques définis de telle façon que les réactions définies ci-dessus soient équilibrées en masse. Selon un mode de mise en oeuvre de la présente invention, les coefficients $a_{SrConsk}$ (avec $1 \leq r \leq 4$ et $1 \leq k \leq 3$) sont estimés en effectuant des bilans de masse sur la distribution du soufre grâce au logiciel Geokin Compo (IFPEN, Energies nouvelles).

### 1.2 Construction d'un modèle cinétique

Au cours de cette étape, on construit un modèle cinétique permettant d'estimer l'évolution de la masse d'hydrogène sulfuré produite par un phénomène d'aquathermolyse. Selon un mode de mise en oeuvre de l'invention, le modèle cinétique peut en outre permettre d'estimer l'évolution au cours du temps de la composition des hydrocarbures du gisement étudié, c'est-à-dire l'évolution au cours du temps de la quantité de chacun des pseudo-constituants de la représentation compositionnelle choisie à l'étape 1.1 précédente.

Le modèle cinétique selon l'invention est défini à partir d'au moins un système de réactions simulant le phénomène d'aquathermolyse, du schéma réactif élémentaire représentatif d'un bilan de matière sur l'élément soufre tel que défini à l'étape 1.1, de bilans atomiques relatifs à chacune des réactions du système de réactions ainsi défini, les bilans portant au moins sur les atomes de soufre, de carbone et d'hydrogène. Par ailleurs, selon l'invention, le système de réactions simulant ledit phénomène d'aquathermolyse est fonction de coefficients stoechiométriques relatifs au moins aux pseudo-constituants définis à l'étape 1.1 et au moins au constituant $H_2S$.

Le modèle cinétique selon l'invention peut être obtenu selon l'enchainement non limitatif des étapes suivantes :

> 1.2.1. Définition d'un système de réactions simulant l'aquathermolyse
> 1.2.2. Définition de bilans atomiques pour chacune des réactions
> 1.2.3. Etablissement d'un lien entre les coefficients pseudo-stoechiométriques du schéma réactif et les coefficients stoechiométriques du système de réactions
> 1.2.4. Détermination des coefficients stoechiométriques du système de réactions

Les étapes 1.2.1 à 1.2.4 de ce mode de réalisation préféré sont détaillées ci-après.

### 1.2.1. Définition d'un système de réactions simulant l'aquathermolyse

Au cours de cette sous-étape, il s'agit de définir un système de réactions décrivant ledit phénomène d'aquathermolyse dans ledit gisement. Selon l'invention, le système de réactions simulant l'aquathermolyse est fonction d'au moins les pseudo-constituants définis à l'étape 1.1 et d'au moins le constituant $H_2S$. De plus, ce système de réactions est exprimé en fonction de coefficients stoechiométriques relatifs à chacun des pseudo-constituants et constituants intervenant dans les réactions.

Selon un mode préféré de mise en oeuvre de la présente invention, chacun des pseudo-constituants définis à l'étape 1.1 est représenté par une pseudo-molécule du type $C_{nC}H_{nH}S_{nS}O_{nO}$, où nC, nH, nS, et nO sont respectivement le nombre d'atomes de carbone, d'hydrogène, de soufre et d'oxygène dans ladite pseudo-molécule. La prise en compte de l'oxygène dans le modèle cinétique permet notamment d'intégrer l'eau ($H_2O$) et le gaz carbonique ($CO_2$) dans le système de réactions simulant l'aquathermolyse. En particulier, la prise en compte de l'eau dans le modèle cinétique permet d'améliorer la précision de la prédiction des bilans massiques, mais aussi de s'assurer que les réactions d'aquathermolyse ne sont simulées que lorsque des molécules d'eau sont bien présentes. Par ailleurs, les pseudo-molécules permettent d'intégrer au modèle cinétique les caractéristiques thermodynamiques moyennes appropriées des pseudo-constituants de la représentation compositionnelle choisie et du ou des pseudo-constituants solides. Afin de déterminer la pseudo-molécule associée à chaque pseudo-constituant, on suppose que l'analyse élémentaire de chaque pseudo-constituant est constante au cours des réactions d'aquathermolyse. On aboutit ainsi à un système de réactions (noté (2) par la suite) de la forme :

$$RES1 + a_{1H2O}\, H_2O \rightarrow a_{1SAT}\, SAT + a_{1ARO}\, ARO + a_{1H2S}\, H_2S + a_{1SLD}\, SLD + a_{1C02}\, C0_2 \quad (r1)$$

$$RES2 + a_{2H2O}\, H_2O \rightarrow a_{2SAT}\, SAT + a_{2ARO}\, ARO + a_{2H2S}\, H_2S + a_{2SLD}\, SLD + a_{2C02}\, C0_2 \quad (r2)$$

$$ASP1 + a_{3H2O}\, H_2O \rightarrow a_{3SAT}\, SAT + a_{3ARO}\, ARO + a_{3H2S}\, H_2S + a_{3SLD}\, SLD + a_{3C02}\, C0_2 \quad (r3)$$

$$ASP2 + a_{4H2O}\, H_2O \rightarrow a_{4SAT}\, SAT + a_{4ARO}\, ARO + a_{4H2S}\, H_2S + a_{4SLD}\, SLD + a_{4C02}\, C0_2 \quad (r4)$$

$$SAT + a_{5H20}\, H_2O \rightarrow a_{5C02}\, C0_2 + a_{5CH4}\, CH_4 \quad (r5)$$

où les $a_{i\,Const\,j}$ sont les coefficients stoechiométriques associés à chaque pseudo-constituant/constituant Const $_{j=\{1:7\}}$={$H_2O$, SAT, ARO, $H_2S$, SLD, $CO_2$, $CH_4$} impliqué dans le système de réactions ainsi défini, avec $a_{i\,Const\,j} \leq 0$ pour les réactants (situés à gauche de la flèche d'une réaction) et $a_{i\,Const\,j} \geq 0$ pour les produits (situés à droite de la flèche d'une réaction) de ce système de réactions. A noter que la cinquième réaction (notée r5) modélise le craquage des saturés en présence de vapeur, ce qui permet (contrairement à la demande de brevet FR 3002969 (US 14/200682) qui ne comporte pas de réaction impliquant les saturés) de prendre en compte la variation de masse de la classe des saturés, variation observée durant des expériences d'aquathermolyse réalisées en laboratoire.

Par ailleurs, chaque réaction chimique du système de réactions selon l'invention est caractérisée par sa propre vitesse de réaction $K_{i=\{1:5\}}$ qui dépend de paramètres dits paramètres cinétiques. Selon un mode de mise en oeuvre de la présente invention, on exprime chaque vitesse de réaction par une loi d'Arrhénius, bien connue de l'homme du métier, de la forme : $K_i = A_i e^{-Ei/RT}$, où $A_i$ est le facteur de fréquence, $E_i$ est l'énergie d'activation, $R$ est la constante des gaz parfaits et $T$ est la température du système.

**1.2.2. Définition de bilans atomiques pour chacune des réactions**

Au cours de cette sous-étape, il s'agit de définir des bilans atomiques au moins sur le carbone (C), sur le soufre (S) et sur l'hydrogène (H) pour chacune des réactions du système de réactions établies à l'étape 1.2.1.

Selon un mode préféré de réalisation de la présente invention, on définit un bilan atomique sur le carbone (C), sur le soufre (S), sur l'hydrogène (H) et sur l'oxygène (O) pour chacune des réactions du système de réactions établi en (2). On aboutit alors à un système d'équations de la forme :

$$n_{C_{Reactant\,i}} = \sum_{j=1}^{j=N} a_{i\,Product\,j}\, n_{C_{Product\,j}}$$

$$n_{S_{Reactant\,i}} = \sum_{j=1}^{j=N} a_{i\,Product\,j}\, n_{S_{Product\,j}} \quad (3)$$

$$n_{H_{Reactant\,i}} + 2\, a_{i\,H20} = \sum_{j=1}^{j=N} a_{i\,Product\,j}\, n_{H_{Product\,j}}$$

$$n_{O_{Reactant\,i}} + a_{i\,H20} = \sum_{j=1}^{j=N} a_{i\,Product\,j}\, n_{O_{Product\,j}} \,,$$

avec l'index i correspondant au numéro de réactions (de i=1 à i=5) du système (2) et Reactant $_{i=\{1:5\}}$={RES1, RES2, ASP1, ASP2, SAT}, l'index j correspondant aux produits de la réaction et Product $_{j=\{1:6\}}$={SAT, ARO, $H_2S$, SLD, $CO_2$, $CH_4$}, et $n_{\{C,S,H,O\}\{Reactant\,i,Product\,j\}}$ correspondant respectivement au nombre d'atomes de carbone, de soufre, d'hydrogène et d'oxygène présents dans le constituant ou le pseudo-constituant considéré.

Selon un mode préféré de réalisation de la présente invention, et à des fins de résolution des systèmes d'équations impliqués dans la présente invention, on exprime en outre le nombre d'atomes de soufre, d'hydrogène et d'oxygène en fonction du nombre d'atomes de carbone pour chacun des pseudo-constituants et constituants considérés dans le système de réactions (2). Pour ce faire, on peut utiliser les ratios S/C, H/C et O/C des pseudo-constituants SARA et SLD (noté $Rx_{/C}$ avec x={S,H,O}), qui sont des informations accessibles à partir de mesures expérimentales réalisées en laboratoire, notamment par une analyse élémentaire des produits d'aquathermolyse simulée en laboratoire. A noter que ces ratios changent légèrement au cours du temps, mais que ces variations peuvent être négligées au premier ordre. A partir de cette hypothèse, on peut écrire un nouveau jeu d'équations de la forme :

$$
\begin{aligned}
n_{S_{Component\,1}} &= Rs_{/C\,Component\,1}\, n_{C_{Component\,1}} \\
n_{H_{Component\,1}} &= R_{H_{/C}\,Component\,1}\, n_{C_{Component\,1}} \\
n_{O_{Component\,1}} &= R_{O_{/C}\,Component\,1}\, n_{C_{Component\,1}}\,,
\end{aligned}
\tag{4}
$$

avec

Component$_{1=\{1:11\}}$={RES1,RES2, ASP1,ASP2, ARO, SAT, SLD, H2O,CO2, H2S, CH4}, $n_{\{S,H,O\}_{Component\,I}}$ étant le nombre d'atomes respectivement de soufre, d'hydrogène, et d'oxygène dans un pseudo-constituant/constituant Component$_{I=\{1:11\}}$, et $n_{C_{Component\,I}}$ étant le nombre d'atomes de carbone dans un pseudo-constituant/constituant Component$_{I=\{1:11\}}$. A noter que les ratios élémentaires H/C et O/C des constituants ($H_2O$, $CO_2$, $H_2S$, $CH_4$) impliqués dans le système de réactions (2) sont immédiats et n'ont pas besoin d'être déterminés par analyse élémentaire.

### 1.2.3. Etablissement d'un lien entre les coefficients pseudo-stoechiométriques du schéma réactif et les coefficients stoechiométriques du système de réactions

Au cours de cette sous-étape, on établit un lien entre les coefficients pseudo-stoechiométriques du schéma réactif du soufre établi à l'étape 1.1 et les coefficients stoechiométriques du système de réactions simulant l'aquathermolyse établi à l'étape 1.2.1 Ce lien peut s'exprimer sous la forme du système d'équations suivant :

$$
\begin{aligned}
a_{i\,H2S} &= a_{S_{iH2S}}\, n_{S_{Reactant\,i}} \\
a_{i\,ARO} &= a_{S_{1ARO}}\, n_{S_{Reactant\,i}} / n_{S_{ARO}} \\
a_{i\,SLD} &= a_{S_{1SLD}}\, n_{S_{Reactant\,i}} / n_{S_{SLD}} \\
a_{S_{iH2S}} &+ a_{S_{iARO}} + a_{S_{iSLD}} = 1
\end{aligned}
\tag{5}
$$

### 1.2.4. Détermination des coefficients stoechiométriques du système de réactions

A cours de cette étape, il s'agit de déterminer une expression des coefficients stoechiométriques du système de réactions (2) en résolvant conjointement les systèmes d'équations (3), (4), et (5).

Selon un mode de mise en oeuvre de la présente invention, il est possible d'utiliser un logiciel de calcul formel, tel que Maple (Maplesoft, Canada), afin d'obtenir une expression des coefficients stoechiométriques du système de réactions (2) en fonction de l'ensemble des variables impliquées dans les systèmes d'équations (3), (4), et (5).

Ainsi, le modèle cinétique construit via le procédé selon l'invention permet de mieux respecter la théorie des phénomènes physiques et chimiques impliqués dans une réaction d'aquathermolyse (notamment le respect des bilans atomiques sur le soufre, mais aussi sur le carbone, l'hydrogène et possiblement l'oxygène, ainsi que la possibilité de faire intervenir l'eau ou le dioxyde de carbone dans le système de réactions), mais aussi de prendre en compte certaines mesures expérimentales disponibles (telles que des analyses élémentaires). Le fait de décrire les pseudo-constituants par des formules moléculaires (des pseudo-molécules) permet d'en déduire directement les propriétés thermodynamiques qui dépendent de ces formules moléculaires, notamment par des corrélations basées sur la masse molaire. La description moléculaire des pseudo-constituants étant également cohérente avec le système de réactions retenu pour décrire les réactions d'aquathermolyse, notamment avec ses coefficients stoechiométriques, on obtient un ensemble cohérent entre les pseudo-formules moléculaires de ces pseudo-constituants, le système de réactions qui décrit l'aquathermolyse en faisant interagir ces pseudo-constituants, et enfin les propriétés thermodynamiques de ces pseudo-constituants.

### 1.3 Construction d'un modèle thermodynamique

Au cours de cette étape, il s'agit de construire un modèle thermodynamique permettant d'estimer les propriétés ou le comportement des phases liquide et/ou vapeur de mélanges de multiples constituants et pseudo-constituants, tels que ceux rencontrés in situ dans les réservoirs de pétrole, de bitume ou de gaz, ou en surface lors de l'exploitation de ces mêmes gisements, et offrant la possibilité de prédire en fonction du temps la composition détaillée de fluides produits au cours de la production.

Dans le contexte réactif de l'invention, il est nécessaire de disposer d'un modèle thermodynamique compositionnel où les compositions des phases non aqueuses et non solides sont détaillées en utilisant les mêmes constituants et pseudo-constituants que ceux impliqués pour la construction du schéma réactif du soufre. Selon un mode de mise en oeuvre de la présente invention, on choisit la masse molaire de chacun des pseudo-constituants identique à celle qui a été utilisée pour construire le système de réactions, et on choisit les autres paramètres thermodynamiques de chacun des constituants par corrélation à leur masse molaire, elle-même déduite de leur pseudo-formule moléculaire. Le ou les solides sont quant à eux uniquement caractérisés par leur masse molaire et ne sont pas considérés dans le calcul des propriétés des phases huile, gaz et eau.

Si l'on choisit d'utiliser une thermodynamique par corrélation, les paramètres des constituants/pseudo-constituants dans les corrélations peuvent être ajustés à partir de calculs effectués avec une équation d'état où les paramètres par constituant/pseudo-constituant sont typiquement obtenus à partir de bases de données quand il s'agit de "corps purs", comme l'$H_2S$ ou, quand il s'agit de pseudo-constituants, à partir de corrélations basées au moins en partie sur la masse molaire.

### 1.4 Calibration du modèle thermo-cinétique

Ainsi, à l'issue des étapes précédentes, on obtient un modèle thermo-cinétique, c'est-à-dire un modèle résultant de la réunion entre le modèle cinétique construit à l'étape 1.2 décrite précédemment et le modèle thermodynamique construit à l'étape 1.3 décrite précédemment. Selon un mode de mise en oeuvre de la présente invention, le modèle thermo-cinétique selon l'invention peut être implémenté dans un simulateur de réservoir, en vue de la calibration du modèle thermo-cinétique.

Au cours de la présente étape, il s'agit de calibrer le modèle thermo-cinétique précédemment construit, c'est-à-dire d'ajuster les paramètres du modèle thermo-cinétique en question de façon à ce que les résultats de simulation numérique du phénomène d'aquathermolyse par le modèle thermo-cinétique soient en accord avec les mesures expérimentales.

Selon un mode de mise en oeuvre de la présente invention, les paramètres à ajuster sont les coefficients pseudo-stoechiométriques du schéma réactif du soufre (décrits à l'étape 1.1) ainsi que les paramètres cinétiques (c'est-à-dire les facteurs de fréquence et les énergies d'activation décrits à l'étape 1.2) du système de réactions du modèle cinétique. Préférentiellement, la calibration du modèle thermo-cinétique consiste en l'ajustement des paramètres cinétiques des cinq réactions du système de réactions défini par l'équation (2) et des coefficients pseudo-stoechiométriques du schéma réactif du soufre défini par l'équation (1).

Selon un autre mode de mise en oeuvre de l'invention, si les coefficients pseudo-stoechiométriques du schéma réactif du soufre ont été préalablement déterminés à partir d'expériences d'aquathermolyse, la calibration des résultats numériques avec les mesures expérimentales est obtenue en ajustant uniquement les paramètres cinétiques des réactions, c'est-à-dire les facteurs de fréquence et les énergies d'activation.

Selon un mode de réalisation de la présente invention, la calibration des paramètres cinétiques des cinq réactions du système de réactions définis par le système (2) et les coefficients pseudo-stoechiométriques du schéma réactif du soufre définis à l'équation (1) est effectuée au moyen d'une technique d'inversion itérative. Plus précisément, à partir de valeurs initiales pour les paramètres à déterminer, on construit une fonction objectif mesurant l'écart entre les quantités normées prédites par les systèmes (1) et (2) pour l'$H_2S$ et les pseudo-constituants de la représentation compositionnelle choisie et leurs mesures expérimentales respectives, puis on modifie les valeurs de ces paramètres, itération après itération, jusqu'à trouver un minimum de la fonction objectif. Lors de la calibration, on privilégie la reproduction des données expérimentales normées concernant l'$H_2S$, car la qualité des prédictions de production d'$H_2S$ en surface, lors de simulations de réservoir impliquant des réactions d'aquathermolyse, dépendent principalement de la bonne prédiction de cette quantité. De nombreux algorithmes de minimisation de fonction objectif sont connus du spécialiste, tels que la méthode de Gauss-Newton, la méthode de Newton-Raphson ou encore le gradient conjugué. Selon un mode de réalisation préféré de la présente invention, on utilise la méthode de Gauss-Newton comme algorithme de minimisation de la fonction objectif précédemment décrite. Un exemple d'un logiciel mettant en oeuvre une technique d'inversion est le logiciel CougarFlow (IFP Energies nouvelles, France), que l'on peut coupler avec le logiciel de simulation de réservoir PumaFlow (IFP Energies nouvelles, France), dans lequel on aura implémenté le modèle thermo-cinétique précédemment défini, pour l'estimation des différents termes de la fonction objectif.

**1.5 Mise en oeuvre de la simulation réservoir**

Au cours de cette étape, il s'agit de déterminer la quantité d'hydrogène sulfuré ($H_2S$) produite au cours du temps par un phénomène d'aquathermolyse en réalisant une simulation de réservoir, appliquée à la représentation maillée du gisement étudié, et au moyen d'un simulateur thermique compositionnel et réactif, ledit simulateur mettant en oeuvre le modèle thermo-cinétique précédemment défini et calibré.

La simulation de réservoir implique de réaliser des calculs d'équilibre de phase et des calculs de propriétés de phase, qui vont être détaillés ci-après.

**1.5.1 Equilibres de phase**

En simulation compositionnelle de réservoir avec présence de vapeur d'eau comme dans le cas étudié, les équilibres de phases entre les phases "liquide aqueux" (dite "eau"), "liquide hydrocarbure" (dite huile), et gaz, se calculent en utilisant typiquement les hypothèses suivantes :

- la phase gaz peut contenir des constituants tels que l'$H_2S$, le $CO_2$ et de la vapeur d'eau, ainsi que des pseudo-constituants de la représentation compositionnelle des hydrocarbures ;

- la phase huile contient tous les pseudo-constituants de la représentation compositionnelle des hydrocarbures, peut contenir de l'$H_2S$ et du $CO_2$, mais ne contient pas d'eau ;

- la phase "eau" est essentiellement de l'eau plus ou moins salée, une option de dissolution dans la phase aqueuse des pseudo-constituants/constituants issus de l'aquathermolyse des hydrocarbures pouvant être activée par exemple pour l'$H_2S$ qui, comme le dioxyde de carbone ($CO_2$), peut se dissoudre notablement dans une phase aqueuse.

Selon un mode de mise en oeuvre de l'invention, le calcul des équilibres entre phases est effectué à partir de constantes d'équilibre par constituant/pseudo-constituant calculées en cours de simulation (ou pré-calculées avant la simulation) à partir de fugacités par constituant/pseudo-constituant par phase, elles-mêmes obtenues à partir d'une équation d'état. Préférentiellement, on utilise une équation d'état cubique :

- pour le partage des constituants/pseudo-constituants entre phases huile et gaz : dans ce cas, l'une des équations les plus couramment utilisées est celle dite de Peng-Robinson, décrite par exemple dans (Peng and Robinson, 1976) ;
- pour le partage des constituants/pseudo-constituants entre phases gaz et eau : dans ce cas l'équation la plus couramment utilisée est celle de Søreide et Whitson décrite par exemple dans (Søreide and Whitson, 1992).

Les logiciels industriels de simulation de réservoir offrent également la possibilité de calculer les équilibres entre phases à partir de constantes d'équilibre tabulées, en fonction de la pression et de la température et possiblement en fonction d'un index compositionnel, introduites par l'ingénieur comme données d'entrées de la simulation.

Une autre possibilité offerte pour les équilibres gaz-huile est que les constantes d'équilibre soient calculées à partir de corrélations analytiques, l'ingénieur ayant alors à introduire les paramètres de chaque constituant/pseudo-constituant dans les corrélations. Ces deux possibilités, constantes d'équilibre tabulées ou par corrélation analytique, sont celles qui sont offertes en premier lieu par les logiciels industriels en contexte réactionnel et thermique ; une description de ces options peut être trouvée dans (Coats, 1980).

Dès lors que les entrées pour le calcul des équilibres sont des constantes d'équilibre par constituant tabulées ou par corrélation, une méthodologie utilisée par les hommes de l'art est de générer les tables ou les paramètres des constituants/pseudo-constituants à partir d'une équation d'état de référence. Les tables doivent être générées pour des pressions et températures pouvant être rencontrées en cours de simulation numérique de réservoir.

Les paramètres des constituants/pseudo-constituants dans l'équation d'état de référence sont typiquement les paramètres critiques (température, pression, volume ou facteur de compressibilité), le facteur acentrique, des paramètres d'interactions binaires entre constituants/pseudo-constituants.

Les paramètres thermodynamiques de corps purs tels que l'$H_2S$ sont connus et sont répertoriés par divers organismes tels que N.I.S.T. (National Institute of Standards and Technology, *http://www.nist.gov*). Par contre, les paramètres de pseudo-constituants, paramètres critiques, facteur acentrique, des paramètres d'interactions binaires doivent être estimés. De nombreuses corrélations sont disponibles, dont des corrélations basées sur la masse molaire du pseudo-constituant, sa densité et sa température d'ébullition, ces deux dernières propriétés pouvant elles-mêmes être estimées par des corrélations basées sur la masse molaire du pseudo-constituant. Pour guider le choix des corrélations à utiliser, on peut utiliser certaines informations relatives à la nature du pseudo-constituant (comme une analyse élémentaire qui donne la répartition en masse de différents éléments atomiques), et/ou à sa structure en s'inspirant par exemple de (Boduszynski, 1987).

Enfin, il convient d'ajouter que la valeur mesurée de la masse molaire de composés lourds est connue pour dépendre de la technique expérimentale utilisée, par exemple comme reporté dans (Merdrignac et Espinat, 2007).

Quelle que soit la sophistication de la méthode utilisée pour les déterminer, les masses molaires des pseudo-constituants lourds restent donc des estimations, qui peuvent être utilisées comme des premières estimations dans un processus d'optimisation de paramètres, notamment du nombre d'atomes de carbone des pseudo-constituants, ou ne pas être modifiées si on les considère comme suffisamment représentatives, ou s'il s'avère a posteriori que les valeurs retenues a priori formaient un choix judicieux.

### 1.5.2 Propriétés de phases

Les propriétés de phases utiles aux calculs effectués en simulation numérique compositionnelle de réservoir sont, par phase : la viscosité, l'enthalpie, la masse molaire, la densité molaire (inverse du volume molaire), le produit de ces deux dernières propriétés étant égal à la masse volumique, dont l'estimation est indispensable aux calculs des effets gravitaires, ceux-ci étant, en effet, liés aux différences de masses volumiques entre phases. Les masses molaires des phases sont calculables directement à partir des résultats des calculs d'équilibre qui fournissent les compositions de chaque phase.

Diverses possibilités sont offertes pour calculer les volumes molaires des phases huile et gaz :

- par une équation d'état typiquement cubique, généralement identique à celle utilisée pour les calculs d'équilibre, en utilisant les mêmes paramètres par constituant que ceux qui sont utilisés pour le calcul des équilibres ;

- par des corrélations différenciées selon la nature de la phase huile ou gaz, ces corrélations utilisant des paramètres spécifiques définis par constituant.

Pour le calcul des viscosités, on peut utiliser une corrélation unique pour les calculs de viscosité des phases huile et gaz ou, plus fréquemment pour la simulation de réservoirs d'huile lourde, une corrélation pour la viscosité de l'huile et une corrélation différente pour la viscosité du gaz. Ces corrélations utilisent des paramètres spécifiques définis par constituant/pseudo-constituant.

Les enthalpies des phases sont habituellement calculées à partir de chaleurs spécifiques définies par constituant/pseudo-constituant et par phase, la chaleur spécifique par constituant/pseudo-constituant dans la phase gaz pouvant être calculée alternativement à partir une chaleur spécifique par constituant/pseudo-constituant dans la phase huile et d'une chaleur latente par constituant/pseudo-constituant. On peut trouver davantage de détails dans la publication de Coats précitée, dans celle de (Crookston, 1979), et dans les manuels de référence de logiciels industriels de simulation de réservoir tels que PumaFlow.

La méthode selon l'invention permet donc de modéliser des fluides hydrocarbonés en un mélange de constituants/pseudo-constituants, chacun de ces constituants/pseudo-constituants étant caractérisé par des paramètres thermodynamiques pour modéliser les propriétés physiques du fluide, cette modélisation thermodynamique étant par ailleurs cohérente avec un modèle cinétique multi-réactionnel, où l'un des produits des réactions modélisées est l'hydrogène sulfuré ($H_2S$).

A l'issue de cette étape, on obtient grâce, à la simulation dite "de réservoir", les quantités d'$H_2S$ pouvant être générées au cours de l'exploitation de gisements pétroliers par injection de vapeur d'eau, ainsi que les variations de la composition de l'huile en surface et dans le réservoir, c'est-à-dire l'évolution au cours du temps de la quantité de chacun desdits pseudo-constituants de la représentation compositionnelle des hydrocarbures choisie à l'étape 1.1.

### 2. Détermination des conditions d'exploitation en fonction de la quantité d'hydrogène sulfuré

Ces quantités d'hydrogène sulfuré peuvent être comparées à une quantité passée mesurée (historique de production). On peut alors ajuster des paramètres du modèle cinétique et/ou du modèle thermique, de façon à ce que les estimations soient plus précises pour le gisement étudié. Avec de tels modèles ajustés, on peut prédire la production d'$H_2S$ du gisement, pour des conditions d'exploitation données.

On peut également déterminer les conditions d'exploitation en adaptant les matériaux de complétion et/ou les dispositifs de traitement de gaz, de façon à limiter les dommages causés par attaque acide.

On peut également modifier les conditions d'injection de la vapeur pour chercher à réduire les quantités d'$H_2S$ produites.

On peut également comparer la quantité d'hydrogène sulfuré à une teneur maximale légale (de 10 à 50 ppm en volume, selon l'organisme suivant : Agency for Toxic Substances & Disease Registry des Etats-Unis), et on détermine alors les conditions d'exploitation de façon à maintenir une production d'hydrogène sulfuré inférieure à cette teneur maximale légale.

### 3. Production des hydrocarbures

En appliquant les conditions d'exploitation déterminée à l'étape 2, par exemple la quantité, le débit, la température de la vapeur injectée, ou le type de matériau, on produit les hydrocarbures dans le respect des normes légales et en minimisant l'impact sur le matériel.

### Produit programme d'ordinateur

En outre, l'invention concerne un produit programme d'ordinateur téléchargeable depuis un réseau de communication et/ou enregistré sur un support lisible par ordinateur et/ou exécutable par un processeur, comprenant des instructions de code de programme pour la mise en oeuvre du procédé tel que décrit précédemment, lorsque ledit programme est exécuté sur un ordinateur.

### Exemple non limitatif d'application

Les caractéristiques et avantages du procédé selon l'invention sont illustrés dans le cadre de l'exploitation de sables bitumineux du champ Fisher Field (projet Foster Creek) en Alberta, province à l'ouest du Canada par une technique dite de SAGD (« Steam-assisted gravity drainage » en anglais).

Pour cet exemple d'application, le procédé selon l'invention est mis en oeuvre avec les hypothèses suivantes :

- on utilise la représentation compositionnelle des hydrocarbures de type SARA, en considérant deux pseudo-constituants pour les résines (RES1, RES2) et deux pseudo-constituants pour les asphaltènes (ASP1, ASP 2) ;

- on considère un seul pseudo-constituant solide SLD ;

- on suppose que chacun des pseudo-constituants est représenté par une pseudo-molécule du type $C_{nC}H_{nH}S_{nS}O_{nO}$ ;

- on utilise le système de réactions (2) pour simuler la réaction d'aquathermolyse sur les sables bitumineux du champ étudié ;

- le modèle cinétique est établi à partir du système de réactions (2) et des systèmes d'équations (3), (4) et (5).

Pour cette application du procédé selon l'invention, des mesures expérimentales ont été préalablement réalisées et sont présentées dans le Tableau 1. Le Tableau 1 résume les résultats issus d'analyses élémentaires réalisées en laboratoire sur des échantillons de sables bitumineux du champ étudié (Lamoureux-Var and Barroux, 2013). Plus précisément, ce tableau présente les valeurs des ratios S/C, H/C obtenus en fin d'aquathermolyse (après 203h), et O/C en début d'aquathermolyse pour les pseudo-constituants SAT, ARO, RES, ASP et SLD.

Le Tableau 2 présente le nombre d'atomes de carbone retenu pour les pseudo-constituants SAT, ARO, RES1, RES 2, ASP1, et ASP2. Ce nombre d'atomes a été choisi à partir d'une banque de données de masses molaires (voir Barroux et al. 2013). Afin de simplifier les équations, les nombres d'atomes de carbone retenus pour les deux pseudo-constituants décrivant la fraction des résines ont été choisis égaux (c'est-à-dire $n_{C_{RES1}}=n_{C_{RES2}}$).

Tableau 1

|  | X=S | X=H | X=O |
|---|---|---|---|
| $R_{X/_C \, SAT}$ | 0.00000 | 1.91090 | 0.00000 |
| $R_{X/_C \, ARO}$ | 0.02172 | 1.38026 | 0.00354 |
| $R_{X/_C \, RES}$ | 0.02263 | 1.29116 | 0.03292 |
| $R_{X/_C \, ASP}$ | 0.02652 | 1.02020 | 0.01001 |

(suite)

|  | X=S | X=H | X=O |
|---|---|---|---|
| $R_{X/C\ SLD}$ | 0.07491 | 2.21299 | 0.00000 |

Tableau 2

| $n_{C_{SAT}}$ | 14 |
|---|---|
| $n_{C_{ARO}}$ | 66 |
| $n_{C_{RES1/2}}$ | 94 |
| $n_{C_{ASP1}}$ | 194 |
| $n_{C_{ASP2}}$ | 256 |

La substitution de ces valeurs expérimentales à la place des variables des équations du système (2) régissant le modèle des réactions d'aquathermolyse permet au système d'équations résultant d'être résolu en fonction des coefficients stoechiométriques du modèle cinétique. Cette résolution est effectuée en utilisant le logiciel de calcul formel Maple. Par construction, les coefficients stoechiométriques des 4 premières réactions du système (2) dépendent seulement des coefficients pseudo-stoechiométriques du schéma réactif du soufre.

Pour les 2 premières réactions cinétiques (c'est-à-dire ri avec i={1,2}), on obtient :

$$a_{i\ H2S}= f(a_{S_{iH2S}})=2.1272\ a_{Si\ H2S}$$

$$a_{i\ SAT}= f(a_{S_{iH2S}},a_{S_{iSLD}})=1.2385+18.9119\ a_{Si\ H2S}+20.3750\ a_{Si\ SLD}$$

$$a_{i\ ARO}= f(a_{S_{iH2S}},a_{S_{iSLD}})=1.4839-1.4839\ a_{Si\ H2S}-1.4839\ a_{Si\ SLD}$$

$$a_{i\ SLD}= f(a_{S_{iSLD}})=31.5522\ a_{Si\ SLD} \qquad (7a)$$

$$a_{i\ H2O}= f(a_{S_{iH2S}},a_{S_{iSLD}})=-(1.2385+18.9119\ a_{Si\ H2S}+20.3750\ a_{Si\ SLD})$$

$$a_{i\ CO2}= f(a_{S_{iH2S}},a_{S_{iSLD}})=1.9931+9.6293\ a_{Si\ H2S}+10.3608\ a_{Si\ SLD}\ ,$$

Pour la réaction r3, on obtient :

$$a_{3\ H2S}= f(a_{S_{3H2S}})=5.1448\ a_{S3\ H2S}$$

$$a_{3\ SAT}= f(a_{S_{3H2S}},a_{S_{3SLD}})=-3.6582+15.2560\ a_{S3\ H2S}+10.2238\ a_{S3\ SLD}$$

$$a_{3\ ARO}= f(a_{S_{3H2S}},a_{S_{3SLD}})=3.5890-3.5860\ a_{S3\ H2S}-3.5890\ a_{S3\ SLD}$$

$$a_{3\ SLD}= f(a_{S_{3SLD}})=76.3120\ a_{S3\ SLD} \qquad (7b)$$

$$a_{3\ H2O}= f(a_{S_{3H2S}},a_{S_{3SLD}})=-(15.5805+45.7402\ a_{S3\ H2S}+49.2787\ a_{S3\ SLD})$$

$$a_{3\ CO2}= f(a_{S_{3H2S}},a_{S_{3SLD}})=8.3419+23.2894\ a_{S3\ H2S}+25.0586\ a_{S3\ SLD}\ ,$$

Pour la réaction r4, on obtient :

$$a_{4\ H2S} = f(a_{S_{4H2S}}) = 6.7891\ a_{S4\ H2S}$$

$$a_{4\ SAT} = f(a_{S_{4H2S}}, a_{S_{4SLD}}) = -4.8273 + 20.1316\ a_{S4\ H2S} + 13.4912\ a_{S4\ SLD}$$

$$a_{4\ ARO} = f(a_{S_{4H2S}}, a_{S_{4SLD}}) = 4.7360 - 4.7360\ a_{S4\ H2S} - 4.7360\ a_{S4\ SLD}$$

$$a_{4\ SLD} = f(a_{S_{4SLD}}) = 100.7004\ a_{S4\ SLD} \tag{7c}$$

$$a_{4\ H2O} = f(a_{S_{4H2S}}, a_{S_{4SLD}}) = -(20.5598 + 60.3582\ a_{S4\ H2S} + 65.0276\ a_{S4\ SLD})$$

$$a_{4\ CO2} = f(a_{S_{4H2S}}, a_{S_{4SLD}}) = 11.0079 + 30.7325\ a_{S4\ H2S} + 33.0671\ a_{S4\ SLD}.$$

Comme les saturés ne contiennent aucun soufre, les coefficients stoechiométriques associés avec la dernière réaction (réaction r5) ne dépendent pas de la matrice des coefficients $[a_{S_{ij}}]$ et sont complétement contraints par le bilan atomique de la réaction 5. On obtient alors :

$$a_{5\ H2O} = -7.3118$$

$$a_{5\ CO2} = 3.6559 \tag{7d}$$

$$a_{5\ CH4} = 10.3441$$

Le modèle cinétique compositionnel ainsi obtenu est ensuite couplé au simulateur de réservoir PumaFlow (IFP Energies nouvelles, France) afin d'effectuer des simulations de réservoir impliquant des réactions d'aquathermolyse. Les mêmes propriétés thermodynamiques ont été choisies pour les 2 pseudo-constituants RES1 et RES2 associés à la fraction résine, soit $MW_{RES1/2} = 1320$ g/mol. Cette hypothèse permet de simplifier le modèle cinétique compositionnel dérivé des équations (3), (4), et (5).

Afin de calibrer le modèle cinétique, on simule en laboratoire des expériences d'aquathermolyse sur un échantillon de sable bitumineux de Fisher Field au Canada (voir par exemple Lamoureux-Var et Lorant (2005a, 2005b)). En parallèle, on établit un modèle pour simuler numériquement ces expériences d'aquathermolyse. Ce modèle (dit « 0D » car il n'y a pas d'écoulement) consiste en une maille qui modélise le réacteur entourée par plusieurs mailles représentant le four utilisé dans les expériences. La maille centrale du réacteur représente le tube expérimental en or dans lequel les réactions d'aquathermolyse ont lieu. L'équation d'état de Peng-Robinson est utilisée pour les simulations de réservoir des expériences d'aquathermolyse en laboratoire. Les résultats numériques sont comparés avec les variations massiques des fractions SARA et de l'$H_2S$ observées expérimentalement. Les résultats concernant les fractions SARA sont exprimés en masse instantanée du composant par masse initiale du composant (gramme par gramme) tandis que la production d'H2S est évaluée en masse d'$H_2S$ par masse initiale totale des composants SARA (gramme par gramme). Cette mise à l'échelle permet de comparer les résultats numériques avec les résultats expérimentaux en se limitant à une reproduction de l'expérience en termes de fraction massique des différents composants.

La calibration des paramètres du modèle thermo-cinétique, à savoir les paramètres cinétiques (facteur de fréquence et énergie d'activation) des cinq réactions du système (2) et les coefficients pseudo-stoechiométriques du schéma réactif du soufre définis en (1) a été effectuée au moyen d'une boucle d'inversion (réalisée avec le logiciel d'inversion CougarFlow (IFP Energies nouvelles)) directement couplée au simulateur de réservoir PumaFlow (IFP Energies nouvelles), sans étape intermédiaire. L'évaluation de la convergence s'effectue au moyen d'une fonction objectif qui calcule les écarts entre les prédictions massiques normées du simulateur et les données expérimentales respectives pour l'$H_2S$, les Saturés, les Aromatiques, les Résines et les Asphaltènes. Afin de reproduire au mieux les émissions d'$H_2S$ rapportées à la masse d'huile, le poids du terme concernant l'$H_2S$ est augmenté par rapport aux autres termes. L'algorithme de Gauss-Newton est ensuite utilisé pour l'optimisation de la fonction objectif. A noter que si les expériences en laboratoire fournissent l'intégralité du schéma réactif du soufre, la calibration du modèle cinétique compositionnel pour simulation de réservoir peut se limiter à la calibration des paramètres cinétiques (facteurs de fréquence et énergies d'activation).

Les valeurs des paramètres cinétiques (facteurs de fréquence et énergies d'activation) et les valeurs des coefficients pseudo-stoechiométriques obtenus après calibration avec les données expérimentales sont résumées dans le Tableau 3. Les coefficients stoechiométriques associés, obtenus grâce au système d'équations (7), pour chacune des réactions r1 à r5 et pour chaque pseudo-constituant et constituant impliqué dans le système d'équations (7), sont rapportés dans le Tableau 4.

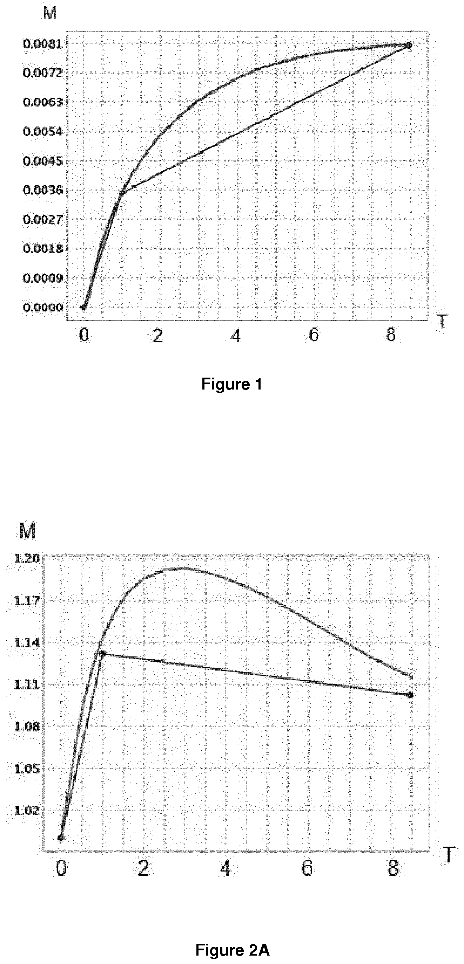

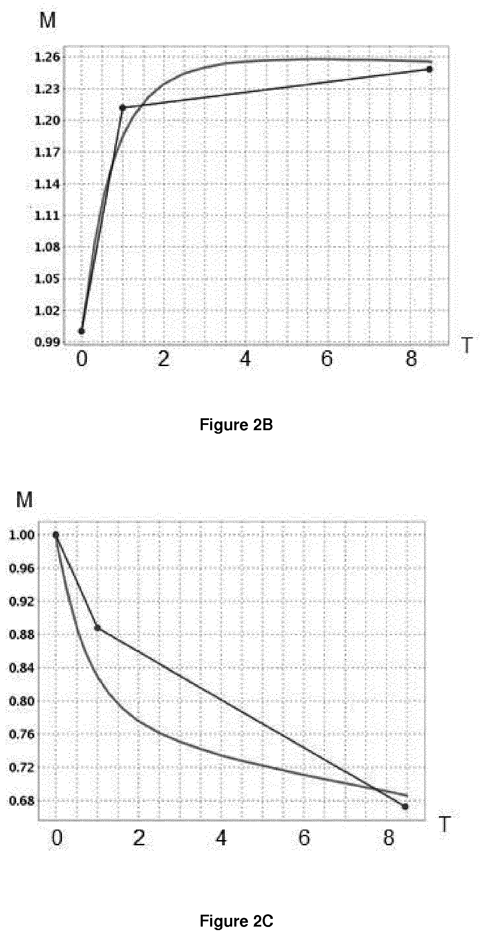

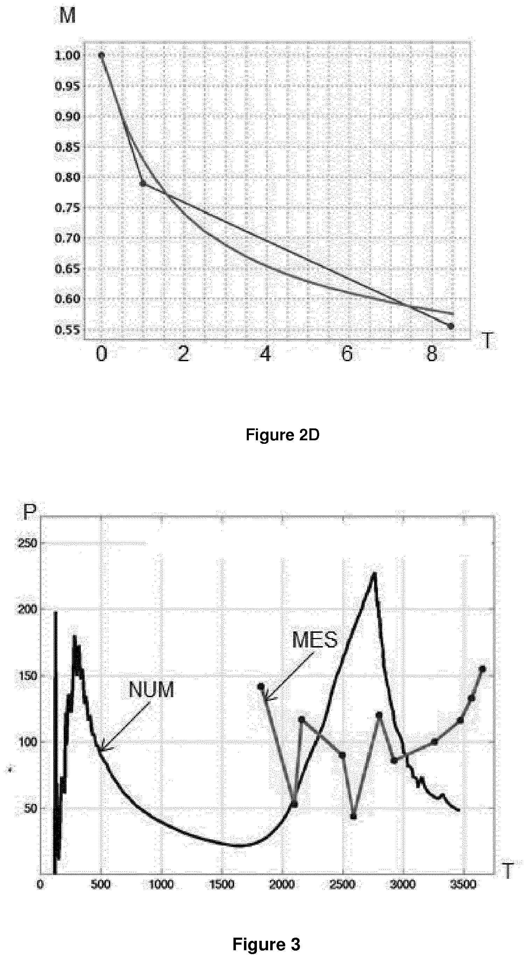

La Figure 1 présente l'évolution au cours du temps T (en jours) de la masse M d'$H_2S$ rapportée à la masse totale initiale des pseudo-constituants SARA, obtenue par la présente invention, mise en oeuvre avec les paramètres précédemment définis (courbe continue), et méthode expérimentale (courbe continue par morceaux). Les Figures 2A, 2B, 2C, et 2D présentent l'évolution au cours du temps T (en jours) de la masse M des pseudo-constituants respectivement SAT, ARO, RESP et ASP rapportée à leur masse initiale, obtenue par la présente invention, mise en oeuvre avec les paramètres précédemment définis (courbes continues), et par méthode expérimentale (courbes continues par morceaux). A la différence des résultats obtenus avec un procédé basé sur la demande de brevet FR 3002969 (US 14/200682), les données expérimentales pour les pseudo-constituants SARA ne sont pas parfaitement reproduites par le modèle cinétique selon l'invention. La raison vient des dépendances introduites entre les différents paramètres de la calibration du modèle cinétique par les équations (3) à (5) précédemment définies. A noter que la demande de brevet FR 3002969 (US 14/200682) permet de bien reproduire les résultats de mesures expérimentales, car la calibration dans cette demande s'affranchissant de nombreuses contraintes théoriques (bilans atomiques) et expérimentales (analyse élémentaire), elle bénéfice de degrés de liberté supplémentaires par rapport à la présente invention. Il n'en reste pas moins que la présente invention permet de reproduire de façon tout à fait satisfaisante les résultats expérimentaux, tout en respectant les paramètres cinétiques et coefficients pseudo-stoechiométriques déterminés lors de l'étape de calibration, ainsi que les contraintes imposées par les équations de bilans atomiques et les données d'analyse élémentaire. Les paramètres présentés dans le Tableau 3 et les coefficients stoechiométriques correspondants, dérivés des équations (7) et rapportés dans le Tableau 4, sont ensuite utilisés pour une simulation de réservoir compositionnelle.

Tableau 3

| Réaction | Facteur de fréquence (Jour$^{-1}$) | Energie d'activation (kJ/mol) | aSH2S | aSSLD |
|----------|-----------------------------------|-------------------------------|-------|-------|
| r1 | 1.00E+19 | 204.30 | 0.369 | 0.000 |
| r2 | 5.00E+18 | 222.22 | 0.899 | 0.151 |
| r3 | 4.50E+18 | 201.34 | 0.651 | 0.349 |
| r4 | 2.00E+18 | 216.67 | 0.960 | 0.040 |
| r5 | 3.82E+18 | 209.09 | | - |

Tableau 4

| Réaction | $H_2O$ | $H_2S$ | SAT | ARO | SLD | $CO_2$ | $CH_4$ |
|----------|--------|--------|-----|-----|-----|--------|--------|
| r1 | -8.22 | 0.78 | 1.90 | 0.94 | 0.00 | 5.55 | - |
| r2 | -21.3 | 1.91 | 5.88 | 0.00 | 4.76 | 12.2 | - |
| r3 | -62.6 | 3.35 | 9.84 | 0.00 | 26.6 | 32.2 | - |
| r4 | -81.1 | 6.52 | 15.0 | 0.00 | 4.03 | 41.8 | - |
| r5 | -7.31 | - | -1 | - | - | 3.66 | 10.34 |

Les caractéristiques du modèle de réservoir utilisé pour modéliser le champ étudié sont présentées dans le Tableau 5. Le domaine du réservoir est rectangulaire avec respectivement des dimensions de 420, 150 et 18 mètres dans les directions X, Y et Z. La grille utilisée pour la simulation est une grille cartésienne de 3375 mailles (1 $\times$ 75 $\times$ 25). Les deux puits horizontaux sont situés au centre de l'axe Y et sont séparés par une distance de 6 mètres suivant l'axe Z. La longueur des puits suivant l'axe X est égale à la largeur du réservoir à savoir 420 mètres. La simulation de réservoir est réalisée dans un coupe verticale perpendiculaire à l'axe Y, caractérisée par une perméabilité et une porosité homogène. Dans les simulations réservoir simulant le procédé SAGD, le système huile-gaz est décrit en utilisant des tables de constantes d'équilibre.

Tableau 5

| | |
|---|---|
| Profondeur du haut du réservoir, m | 300 |
| Dimensions totales dans les directions X,Y,Z, m | 420, 150, 18 |
| Perméabilité, horizontale, verticale, mD | 10 000, 3 |
| Porosité | 0.37 |

(suite)

| Pression initiale @300 m, bar | 29 |
|---|---|
| Temperature initiale, °C | 17.0 |
| Densité de l'huile, API | 10 |
| Viscosité de l'huile, cp | 3.8E+06 |
| Saturation initiale de l'eau | 0.22 |
| Fraction SAT initiale, fraction molaire | 0.60752 |
| Fraction ARO initiale, fraction molaire | 0.14992 |
| Fraction RES1 initiale, fraction molaire | 0.04071 |
| Fraction RES2 initiale, fraction molaire | 0.14499 |
| Fraction ASP1 initiale, fraction molaire | 0.01871 |
| Fraction ASP2 initiale, fraction molaire | 0.03815 |

La pression initiale est de 29 bars en haut du réservoir, lequel est situé à une profondeur de 300 mètres. La composition initiale du fluide est résumée dans le Tableau 5 et provient de mesures expérimentales sur le champ étudié. Plus de détails relatifs à la caractérisation de l'huile et aux perméabilités relatives utilisées sont disponibles dans (Barroux et al., 2013). Avant de commencer l'injection de vapeur, les puits sont préchauffés pendant une période de 4 mois pour mobiliser l'huile à proximité.

La Figure 3 représente la production P simulée d'$H_2S$ en fonction du temps T (en jours), exprimée en litres d'$H_2S$ produits par $m^3$ d'huile produite en surface, obtenue par la présente invention (courbe notée NUM) mise en oeuvre avec les paramètres et hypothèses précédemment définis. Sur cette figure, la production d'$H_2S$, observée in situ, est également représentée (courbe notée MES). Elle est déduite de mesures réalisées entre 2005 et 2012 sur le champ étudié, mesures publiées par les compagnies pétrolières Encana et Cenovus, sur le site web de l'Energy Resources Conservation Board (2012). Cette figure montre que la présente invention, mise en oeuvre avec les paramètres et hypothèses précédemment définis, ne permet pas de reproduire exactement les données expérimentales de production. Ceci s'explique par le fait que, d'une part le modèle de réservoir utilisé est extrêmement simplifié (la porosité et la perméabilité sont supposées homogènes et les paramètres génériques utilisés pour modéliser le réservoir de sable bitumineux d'Athabasca peuvent présenter des écarts avec les paramètres spécifiques du champs étudié), et d'autre part, on a limité la simulation de réservoir à deux puits, alors que les données de production observées proviennent d'un champ qui comprend des dizaines de puits (les données de production ayant été ramenées en moyenne à celle obtenues pour un puits unique pour les besoins de l'exemple). Néanmoins les résultats obtenus avec une mise en oeuvre de l'invention sont du même ordre de grandeur (de l'ordre de la centaine) que les données de production, ce qui permet de valider la présente invention à l'échelle du réservoir.

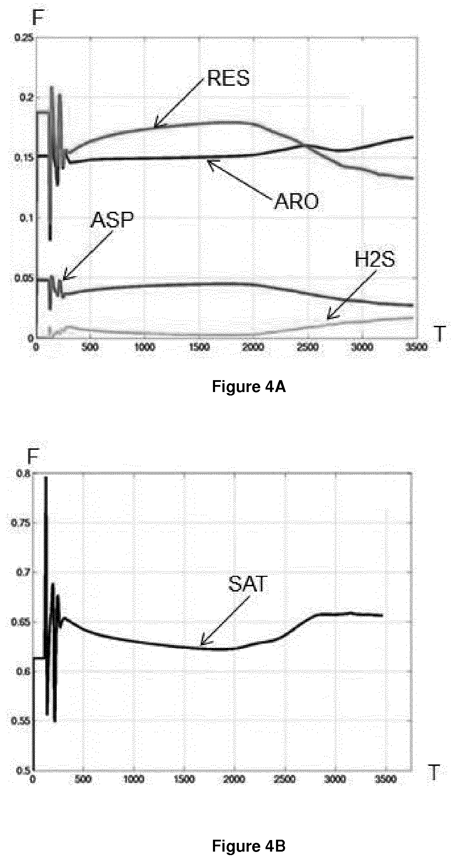

Les Figures 4A et 4B montrent l'évolution en fonction du temps de la composition d'huile produite en condition de surface. En particulier, la Figure 4A présente l'évolution de la fraction molaire F des pseudo-constituants ASP, ARO, RES et du constituant H2S au cours du temps T (en jours), et la Figure 4B présente l'évolution de la fraction molaire F du pseudo-constituant SAT au cours du temps T (en jours). A partir de l'observation de ces figures, il apparaît clairement que la composition de l'huile change entre le début de la production et la fin de la simulation, presque 10 années plus tard. En particulier, la quantité de saturés (SAT) et d'aromatiques (ARO) dans l'huile produite a globalement augmenté, tandis que la quantité de résines (RES) et d'asphaltènes (ASP) a globalement diminué, ce qui est cohérent avec les résultats expérimentaux précédemment rapportés dans la littérature concernant ce champ (par exemple Belgrave et al, 1997).

Ainsi, la présente invention permet de mieux respecter la théorie représentative des phénomènes physiques et chimiques impliqués dans une réaction d'aquathermolyse (notamment le respect des bilans atomiques du soufre, mais aussi de carbone, de l'hydrogène et possiblement de l'oxygène ; la possibilité de faire intervenir l'eau dans le système de réactions), mais aussi de prendre en compte certaines mesures expérimentales disponibles (telles que des analyses élémentaires) dans l'élaboration du mécanisme réactionnel. Pour ce faire, les coefficients stoechiométriques associés aux réactions sont exprimés en termes de fraction molaire. Ce modèle cinétique assure également la cohérence entre les paramètres thermodynamiques des constituants, leur pseudo-formule moléculaire et le schéma réactif.

**Revendications**

1. Procédé d'exploitation d'un gisement souterrain d'hydrocarbures contenant des composés organo-soufrés, **caractérisé en ce que** :

    A. on détermine une quantité d'hydrogène sulfuré ($H_2S$) produite au cours du temps par un phénomène d'aquathermolyse induit par un procédé thermique tel qu'une injection de vapeur d'eau dans ledit gisement, à partir d'une représentation maillée dudit gisement, à partir de mesures expérimentales réalisées sur au moins un échantillon desdits hydrocarbures et/ou un échantillon de roche dudit gisement, en mettant en oeuvre les étapes suivantes :

    i. on construit un schéma réactif élémentaire représentatif d'un bilan de matière sur l'élément soufre à partir d'au moins une représentation compositionnelle des hydrocarbures, ladite représentation décrivant lesdits hydrocarbures en différents pseudo-constituants, d'au moins un pseudo-constituant solide, d'au moins un constituant $H_2S$, et de coefficients pseudo-stoechiométriques relatifs auxdits pseudo-constituants et constituants ;
    ii. on construit un modèle cinétique à partir d'au moins un système de réactions simulant ledit phénomène d'aquathermolyse, ledit schéma réactif, de bilans atomiques relatifs à chacune desdites réactions dudit système de réactions, lesdits bilans portant au moins sur les atomes de soufre, de carbone et d'hydrogène, et ledit système de réactions étant fonction de coefficients stoechiométriques relatifs au moins auxdits pseudo-constituants et constituants ;
    iii. on construit un modèle thermodynamique des pseudo-constituant de ladite représentation compositionnelle ;
    iv. on calibre un modèle thermo-cinétique, constitué dudit modèle cinétique et dudit modèle thermodynamique, à partir desdites mesures expérimentales ;
    v. on détermine ladite quantité d'hydrogène sulfuré ($H_2S$) produite en réalisant une simulation de réservoir compositionnelle, au moyen d'un simulateur thermique compositionnel et réactif, ledit simulateur mettant en oeuvre ledit modèle thermo-cinétique, et au moyen de ladite représentation maillée ;

    B. on détermine des conditions d'exploitation dudit gisement en fonction de ladite quantité d'hydrogène sulfuré ;
    C. on produit lesdits hydrocarbures en appliquant lesdites conditions d'exploitation.

2. Procédé selon la revendication précédente, dans lequel ladite représentation compositionnelle des hydrocarbures est constituée des pseudo-constituants suivants : les hydrocarbures saturés, les hydrocarbures aromatiques, les résines et les asphaltènes.

3. Procédé selon l'une des revendications précédentes, dans lequel lesdites mesures expérimentales consistent en des expériences d'aquathermolyse simulées en laboratoire.

4. Procédé selon l'une des revendications précédentes, dans lequel l'étape ii) est réalisée selon les étapes suivantes :

    a) on définit un système de réactions décrivant ledit phénomène d'aquathermolyse dans ledit gisement, ledit système étant fonction au moins desdits pseudo-constituants et au moins des constituants $H_2S$ et $H_2O$, et desdits coefficients stoechiométriques relatifs à chacun desdits pseudo-constituants et constituants ;
    b) on définit un premier système d'équations établissant, pour chacune desdites réactions, des bilans atomiques pour les éléments atomiques H, C, S et O ;
    c) on définit un deuxième système d'équations reliant lesdits coefficients pseudo-stoechiométriques dudit schéma réactif auxdits coefficients stoechiométriques dudit système de réactions, la somme desdits coefficients pseudo-stoechiométriques dudit schéma réactif étant égales à 1 ;
    d) on détermine une expression desdits coefficients stoechiométriques dudit système de réactions en résolvant conjointement lesdits systèmes d'équations.

5. Procédé selon la revendication 4, dans lequel on définit un troisième système d'équations reliant le nombre d'atomes de soufre, de carbone et d'oxygène en fonction du nombre d'atomes de carbone pour chacun desdits pseudo-constituants et constituants.

6. Procédé selon la revendication 5, dans lequel on relie ledit nombre d'atomes de soufre, de carbone et d'oxygène en fonction dudit nombre d'atomes de carbone pour chacun desdits pseudo-constituants via des ratios déterminés par des analyses élémentaires réalisées sur des produits d'aquathermolyse simulées en laboratoire.

7. Procédé selon l'une des revendications précédentes, dans lequel à l'étape iii), on calibre au moins des paramètres cinétiques dudit modèle thermo-cinétique.

8. Procédé selon l'une des revendications 1 à 6, dans lequel à l'étape iii), on calibre des paramètres cinétiques dudit modèle thermo-cinétique et lesdits coefficients pseudo-stoechiométriques dudit schéma réactif.

9. Procédé selon l'une des revendications précédentes, dans lequel on détermine en sus à l'étape A) une évolution au cours du temps de la quantité de chacun desdits pseudo-constituants de ladite représentation compositionnelle.

10. Procédé selon l'une des revendications précédentes, dans lequel on détermine lesdites conditions d'exploitation en adaptant des matériaux de complétion et/ou des dispositifs de traitement de gaz en fonction de ladite quantité d'hydrogène sulfuré.

11. Procédé selon l'une des revendications précédentes, dans lequel lesdites conditions d'exploitation consistent à modifier des conditions d'injection de la vapeur de façon à minimiser ladite quantité d'hydrogène sulfuré.

12. Procédé selon l'une des revendications précédentes, dans lequel on détermine lesdites conditions d'exploitation de façon à maintenir une production d'hydrogène sulfuré inférieure à une teneur maximale légale.

13. Produit programme d'ordinateur téléchargeable depuis un réseau de communication et/ou enregistré sur un support lisible par ordinateur et/ou exécutable par un processeur, comprenant des instructions de code de programme pour la mise en oeuvre du procédé selon l'une des revendications précédentes, lorsque ledit programme est exécuté sur un ordinateur.

**Patentansprüche**

1. Verfahren zur Nutzung einer unterirdischen Lagerstätte von Kohlenwasserstoffen, enthaltend Organoschwefelverbindungen, **dadurch gekennzeichnet, dass**:

A. eine Menge an Schwefelwasserstoff ($H_2S$), die mit der Zeit durch ein Aquathermolysephänomen erzeugt wird, das durch einen thermischen Prozess induziert wird, wie ein Einbringen von Wasserdampf in die Lagerstätte, aus einer Gitterdarstellung der Lagerstätte durch experimentelle Messungen bestimmt wird, die an mindestens einer Probe der Kohlenwasserstoffe und/oder einer Gesteinsprobe der Lagerstätte durchgeführt werden, indem die folgenden Schritte durchgeführt werden:

i. ein Elementarreaktionsschema wird konstruiert, das für eine Stoffbilanz an dem Element Schwefel aus mindestens einer Zusammensetzungsdarstellung der Kohlenwasserstoffe repräsentativ ist, wobei die Darstellung die Kohlenwasserstoffe in verschiedenen Pseudobestandteilen, mindestens einem festen Pseudobestandteil, mindestens einem $H_2S$-Bestandteil und pseudostöchiometrischen Koeffizienten in Bezug auf die Pseudobestandteile und Bestandteile beschreibt;
ii. ein kinetisches Modell wird aus mindestens einem Reaktionssystem konstruiert, welches das Aquathermolysephänomen und das Reaktionsschema der Atombilanzen in Bezug auf jede der Reaktionen des Reaktionssystems simuliert, wobei die Bilanzen mindestens die Schwefel-, Kohlenstoff- und Wasserstoffatome betreffen, und das Reaktionssystem eine Funktion der stöchiometrischen Koeffizienten mindestens in Bezug auf die Pseudobestandeile und Bestandteile ist;
iii. ein thermodynamisches Modell des Pseudobestandteils der Zusammensetzungsdarstellung wird konstruiert;
iv. ein thermokinetisches Modell, bestehend aus dem kinetischen Modell und dem thermodynamischen Modell, wird aus den experimentellen Messungen kalibriert;
v. die Menge an Schwefelwasserstoff ($H_2S$), die mittels der Durchführung einer Simulation des Zusammensetzungsvorkommens erzeugt wird, wird mit einem thermischen Zusammensetzungs- und Reaktionssimulator, wobei der Simulator das thermokinetische Modell verwendet, und mit der Gitterdarstellung bestimmt;

B. die Nutzungsbedingungen der Lagerstätte werden als Funktion der Menge an Schwefelwasserstoff bestimmt;

C. die Kohlenwasserstoffe werden unter Anwendung der Nutzungsbedingungen erzeugt.

2. Verfahren nach dem vorhergehenden Anspruch, wobei die Zusammensetzungsdarstellung der Kohlenwasserstoffe aus den folgenden Pseudobestandteilen besteht: gesättigten Kohlenwasserstoffen, aromatischen Kohlenwasserstoffen, Harzen und Asphaltenen.

3. Verfahren nach einem der vorhergehenden Ansprüche, wobei die experimentellen Messungen aus im Labor simulierten Aquathermolyseversuchen bestehen.

4. Verfahren nach einem der vorhergehenden Ansprüche, wobei der Schritt ii) gemäß den folgenden Schritten durchgeführt wird:

a) ein Reaktionssystem wird definiert, welches das Aquathermolysephänomen in der Lagerstätte beschreibt, wobei das System eine Funktion mindestens der Pseudobestandteile und mindestens der Bestandteile $H_2S$ und $H_2O$ und der stöchiometrischen Koeffizienten in Bezug auf jeden der Pseudobestandteile und Bestandteile ist;
b) ein erstes Gleichungssystem wird definiert, das für jede der Reaktionen Atombilanzen für die Atomelemente H, C, S und O festlegt;
c) ein zweites Gleichungssystem wird definiert, das die pseudostöchiometrischen Koeffizienten des Reaktionsschemas mit den stöchiometrischen Koeffizienten des Reaktionssystems verbindet, wobei die Summe der pseudostöchiometrischen Koeffizienten des Reaktionsschemas gleich 1 ist;
d) ein Ausdruck der stöchiometrischen Koeffizienten des Reaktionssystems wird bestimmt, indem die Gleichungssysteme gemeinsam gelöst werden.

5. Verfahren nach Anspruch 4, wobei ein drittes Gleichungssystem definiert wird, das die Anzahl von Schwefel-, Kohlenstoff- und Sauerstoffatomen als Funktion der Anzahl von Kohlenstoffatomen für jeden der Pseudobestandteile und Bestandteile verbindet.

6. Verfahren nach Anspruch 5, wobei die Anzahl von Schwefel-, Kohlenstoff- und Sauerstoffatomen als Funktion der Anzahl von Kohlenstoffatomen für jeden der Pseudobestandteile und Bestandteile über Verhältnisse verbunden wird, die durch elementare Analysen bestimmt werden, welche an im Labor simulierten Aquathermolyseprodukten durchgeführt werden.

7. Verfahren nach einem der vorhergehenden Ansprüche, wobei in dem Schritt iii) mindestens die kinetischen Parameter des thermokinetischen Modells kalibriert werden.

8. Verfahren nach einem der Ansprüche 1 bis 6, wobei in dem Schritt iii) die kinetischen Parameter des thermokinetischen Modells und die pseudostöchiometrischen Koeffizienten des Reaktionsschemas kalibriert werden.

9. Verfahren nach einem der vorhergehenden Ansprüche, wobei zusätzlich zu Schritt A) eine Entwicklung der Menge jedes der Pseudobestandteile der Zusammensetzungsdarstellung mit der Zeit bestimmt wird.

10. Verfahren nach einem der vorhergehenden Ansprüche, wobei die Nutzungsbedingungen bestimmt werden, indem Komplettierungsmaterialien und/oder Gasbehandlungsvorrichtungen als Funktion der Menge an Schwefelwasserstoff angepasst werden.

11. Verfahren nach einem der vorhergehenden Ansprüche, wobei die Nutzungsbedingungen aus der Modifikation der Bedingungen zum Einbringen des Dampfes bestehen, um die Menge an Schwefelwasserstoff zu minimieren.

12. Verfahren nach einem der vorhergehenden Ansprüche, wobei die Nutzungsbedingungen derart bestimmt werden, dass eine Schwefelwasserstoffproduktion unter einem maximalen legalen Gehalt gehalten wird.

13. Computerprogrammprodukt, welches von einem Kommunikationsnetz heruntergeladen werden kann oder auf einem computerlesbaren Medium aufgezeichnet werden kann und/oder von einem Prozessor ausgeführt werden kann, umfassend Programmcodeinstruktionen zur Durchführung des Verfahrens nach einem der vorhergehenden Ansprüche, wenn das Programm auf einem Computer ausgeführt wird.

**Claims**

1. A method of developing an underground reservoir of hydrocarbons containing organosulfur compounds, **characterized in that** it comprises:

    A. determining an amount of hydrogen sulfide ($H_2S$) produced over time by an aquathermolysis phenomenon induced by a thermal process such as steam injection into said reservoir, from a gridded representation of said reservoir, from experimental measurements performed on at least one sample of said hydrocarbons and/or a rock sample from said reservoir, by carrying out the following steps:

    i. constructing an elementary reaction scheme representative of a material balance for the element sulfur from at least one compositional representation of the hydrocarbons, said representation describing said hydrocarbons in different pseudo-constituents, of at least one solid pseudo-constituent, at least one $H_2S$ constituent and pseudo-stoichiometric coefficients relative to said pseudo-constituents and constituents;
    ii. constructing a kinetic model from at least one reaction system simulating said aquathermolysis phenomenon, said reaction scheme, of atomic balances relative to each one of said reactions of said reaction system, said balances relating at least to the sulfur, carbon and hydrogen atoms, and said reaction system being a function of stoichiometric coefficients relative to at least said pseudo-constituents and constituents;
    iii. constructing a thermodynamic model of the pseudo-constituents of said compositional representation;
    iv. calibrating a thermo-kinetic model consisting of said kinetic model and of said thermodynamic model, from said experimental measurements;
    v. determining said amount of hydrogen sulfide ($H_2S$) produced by performing a compositional reservoir simulation by means of a compositional and reactive thermal simulator, said simulator implementing said thermo-kinetic model, and by means of said gridded representation;

    B. determining development conditions for said reservoir as a function of said amount of hydrogen sulfide;
    C. producing said hydrocarbons by applying said development conditions.

2. A method as claimed in the previous claim, wherein said compositional representation of the hydrocarbons consists of the following pseudo-constituents: saturated hydrocarbons, aromatic hydrocarbons, resins and asphaltenes.

3. A method as claimed in any one of the previous claims, wherein said experimental measurements consist of aquathermolysis experiments simulated in the laboratory.

4. A method as claimed in any one of the previous claims, wherein step ii) is carried out according to the following steps:

    a) defining a reaction system describing said aquathermolysis phenomenon in said reservoir, said system being a function of at least said pseudo-constituents and at least constituents $H_2S$ and $H_2O$, and of said stoichiometric coefficients relative to each one of said pseudo-constituents and constituents;
    b) defining a first system of equations establishing, for each one of said reactions, atomic balances for the atomic elements H, C, S and O;
    c) defining a second system of equations connecting said pseudo-stoichiometric coefficients of said reaction scheme to said stoichiometric coefficients of said reaction system, the sum of said pseudo-stoichiometric coefficients of said reaction scheme being 1;
    d) determining an expression for said stoichiometric coefficients of said reaction system by solving said systems of equations together.

5. A method as claimed in claim 4, comprising defining a third system of equations connecting the number of sulfur, carbon and oxygen atoms as a function of the number of carbon atoms for each one of said pseudo-constituents and constituents.

6. A method as claimed in claim 5, comprising connecting said number of sulfur, carbon and oxygen atoms as a function of said number of carbon atoms for each one of said pseudo-constituents via ratios determined through elementary analyses performed on laboratory-simulated aquathermolysis products.

7. A method as claimed in any one of the previous claims wherein, in step iii), at least kinetic parameters of said thermo-kinetic model are calibrated.

8. A method as claimed in any one of claims 1 to 6 wherein, in step iii), kinetic parameters of said thermo-kinetic model and said pseudo-stoichiometric coefficients of said reaction scheme are calibrated.

9. A method as claimed in any one of the previous claims, wherein an evolution over time of the amount of each one of said pseudo-constituents of said compositional representation is additionally determined in step A).

10. A method as claimed in any one of the previous claims, wherein said development conditions are determined by adapting completion materials and/or gas treatment devices as a function of said amount of hydrogen sulfide.

11. A method as claimed in any one of the previous claims, wherein said development conditions consist in modifying steam injection conditions so as to minimize said amount of hydrogen sulfide.

12. A method as claimed in any one of the previous claims, wherein said development conditions are determined so as to maintain a hydrogen sulfide production below a legal maximum amount.

13. A computer program product downloadable from a communication network and/or recorded on a computer readable medium and/or processor executable, comprising program code instructions for implementing the method as claimed in any one of the previous claims, when said program is executed on a computer.

Figure 1

Figure 2A

Figure 2B

Figure 2C

Figure 2D

Figure 3

**Figure 4A**

**Figure 4B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2892817
- US 11588365 B
- FR 3002969
- US 14200682 B
- FR 289281

### Littérature non-brevet citée dans la description

- **BARROUX, C. ; LAMOUREUX-VAR, V. ; FLAU-RAUD, E.** Forecasting of H2S Production due to Aquathermolysis Reactions. *Paper SPE 164317, presented at the SPE Middle East Oil and Gas Show and Conference,* 2013
- **BELGRAVE J.D.M. ; MOORE R.G. ; URSENBACH R.G.** Comprehensive kinetic models for the aquathermolysis of heavy oils. *Journal of Canadian Petroleum Technology,* 1997, vol. 36 (4), 38-44
- **BODUSZYNSKI, M.M.** Composition of Heavy Petroleums. 1. Molecular Weight, Hydrogen Deficiency, and Heteroatom Concentration as a Function of Atmospheric Equivalent Boiling Point up to 1400 °F (760 °C). *Energy & Fuels,* 1987, vol. 1, 2-11
- **COATS, K. H.** In-Situ Combustion Model. *SPE Journal,* 1980, 533-554
- **MERDRIGNAC, I. ; ESPINAT D.** Physicochemical Characterization of Petroleum Fractions: the State of the Art. *Oil & Gas Science and Technology - Rev. IFP,* 2007, vol. 62 (1), 7-32
- **CROOKSTON, R. B. ; CULHAM, W. E. ; CHEN, W. H.** A Numerical Simulation Model Recovery Processes for Thermal Recovery Processes. *SPE 6724, SPE J.,* 1979, 37-58
- **FAN T. ; BUCKLEY, J.** Rapid and Accurate SARA Analysis of Medium Gravity Crude Oils. *Energy Fuels,* 2002, vol. 16 (6), 1571-1575
- **LAMOUREUX-VAR, V. ; LORANT, F. ; BARROUX, C.** Using Geochemistry to Address H2S Artificial Formation as a Result of Production Risk due to Steam Injection for EOR: a Compositional Kinetic Approach. *Oil Sands. Paper 13HOCC-P-412-SPE 97810, SPE/PS-CIM/CHOA International Thermal Operations and Heavy Oil Symposium, 1-3 NovemberConference,* 11 Juin 2005
- **LAMOUREUX-VAR, V. ; LORANT, F.** Expérimental evaluation of H2S yields in reservoir rocks submitted to steam injection. *Paper D08, 13th European Symposium on Improved Oil Recovery,* 25 Avril 2005
- **PENG, D. Y. ; ROBINSON, D. B.** *A New Two-Constant Equation of State. Industrial and Engineering Chemistry Fundamentals,* 1976, vol. 15, 59-64
- **SØREIDE, I. ; WHITSON, C. H.** Peng-Robinson Predictions for Hydrocarbons CO2, N2, and H2S with Pure Water and NaCl Brine. *Fluid Phase Equilibria,* 1992, vol. 77, 217-240